# EUROPEAN PATENT APPLICATION

(11) **EP 2 481 418 A1**
(43) Date of publication of application: **01.08.2012**
(21) Application number: 12165994.0
(22) Date of filing: 15.02.2008
(51) Int. Cl.: A61K 39/00, A61K 39/145

(54) **A method for enhancing T cell response**

(30) Priority: 15.02.2007 US 901980 P
(62) Divisional of application: 08725654.1
(71) Applicant: MannKind Corporation, Valencia, CA 91355 (US)
(72) Inventor: Kundig, Thomas, 8303 Bassersdorf (CH); Bot, Adrian, Valencia, CA 91354 (US); Smith, Kent Andrew, Ventura, CA 93001 (US); Qiu, Zhiyong, San Gabriel, CA 91776 (US)
(74) Representative: Lasar, Andrea Gisela

(57) **Abstract**

Embodiments of the invention disclosed herein relate to methods and compositions for exponentially increasing antigenic stimulation of class I MHC CD8⁺ T cell responses over that based in the art. Some embodiments relate to an immunogenic composition that enhances an immune response in a subject. In some embodiments, the immunogenic composition comprises an antigen in combination with an immunopotentiator or a biological response modifier (BRM). Overall, the invention disclosed herein demonstrates that increasing antigenic stimulation in a manner independent of the dose of the antigen enhances immunogenicity.

SEQUENCE LISTING

<110> Mannkind Corporation

<120> A METHOD FOR ENHANCING T CELL RESPONSE

<130> M 10380/RN

<150> US 60/901,980
<151> 2007-02-15

<160> 8

<170> FastSEQ for Windows Version 4.0

<210> 1
<211> 9
<212> PRT
<213> Artificial Sequence

<220>
<223> Synthesized peptide

<400> 1
Gly Ile Leu Gly Phe Val Phe Thr Leu
 1               5

<210> 2
<211> 9
<212> PRT
<213> Artificial Sequence

<220>
<223> Synthesized peptide

<400> 2
Arg Ala His Tyr Asn Ile Val Thr Phe
 1               5

<210> 3
<211> 9
<212> PRT
<213> Artificial Sequence

<220>
<223> Synthesized peptide

<400> 3
Lys Ala Val Tyr Asn Phe Ala Thr Met
 1               5

<210> 4
<211> 20
<212> PRT
<213> Artificial Sequence

<220>
<223> Synthesized peptide

<400> 4
Gly Leu Asn Gly Pro Asp Ile Tyr Lys Gly Val Tyr Gln Phe Lys Ser
1               5                   10                  15
Val Glu Phe Asp
            20

<210> 5
<211> 14
<212> PRT
<213> Artificial Sequence

<220>
<223> Synthesized peptide

<400> 5
Ser Asp Leu Arg Gly Tyr Val Tyr Gln Gly Leu Lys Ser Gly
1               5                   10

<210> 6
<211> 9
<212> PRT
<213> Artificial Sequence

<220>
<223> Synthesized peptide

<400> 6
Gly Ile Leu Gly Phe Val Phe Thr Leu
1               5

<210> 7
<211> 9
<212> PRT
<213> Artificial Sequence

<220>
<223> Synthesized peptide

<400> 7
Arg Ala His Tyr Asn Ile Val Thr Phe
1               5

<210> 8
<211> 21
<212> DNA
<213> Artificial Sequence

<220>
<223> Synthesized nucleotide sequence

<400> 8
tccatgacgt tcctgaataa t                                              21

## Description

### CROSS-REFERENCE TO RELATED APPLICATIONS

This application claims priority from U.S. Provisional Application Serial No. 60/901,980 filed on February 15, 2007, which is incorporated herein by reference in its entirety.

### FIELD OF THE INVENTION

Embodiments of the invention relate to the fields of immunology and vaccine development. Embodiments of the invention disclosed herein relate to methods and compositions for enhancing immunization and vaccination. More particularly, embodiments of the present invention relate to a method of improving the stimulation of T cell responses. Some embodiments of the invention have further utility as a vaccination strategy in treating diseases such as infectious diseases or cancer.

### BACKGROUND

Live attenuated vaccines usually induce strong and long lasting immune responses after one injection, and many viral vaccines of this type have efficiencies greater than 90% (Nossal, G. Vaccines *in* Fundamental Immunology (*ed*., Paul, W.E.) 1387-1425; Lippincot-Raven Publishers, Philadelphia, 1999, which is herein incorporated by reference in its entirety). In contrast, vaccines consisting of killed microorganisms, toxins, subunit vaccines including peptide vaccines, or naked DNA vaccines, are of considerably lower efficacy, and boosting immunizations are essential. While live vaccines produce increasing antigen doses that call for strong immune responses, non-replicating vaccines produce a decreasing antigen profile that is, as demonstrated in the examples herein, a rather weak stimulus for T cells.

A continuing need exists to develop immunization models that enhance T cell responses against diseases such as, but not limited to, infectious diseases or cancer. Thus, embodiments of the invention disclosed herein relate to an immunotherapeutic approach involving increasing antigenic stimulation over the course of immunization, independent of the cumulative total antigen dose, to enhance immunogenicity. Thus, embodiments of the invention disclosed herein provide for a revision of current immunization models and for methods and compositions for the design and use of vaccines and immunotherapies.

### SUMMARY OF THE INVENTION

Embodiments of the invention disclosed herein relate to methods and compositions for optimizing CD8⁺ T cell responses. Therefore, some embodiments of the invention relate to methods for stimulating a class I MHC-restricted T cell response in a mammal; the method comprises administering a plurality of sequential doses of an immunogenic composition to the mammal wherein each dose subsequent to the initial dose is greater than the immediately preceding dose.

In some embodiments, the sequential doses increase as a linear function of the initial dose. In yet another embodiment, the sequential doses increase as an exponential function of the initial dose. The exponential function is defined by an exponential factor ≥ 2ⁿ⁻¹. In further embodiments, the exponential factor is 5ⁿ⁻¹.

In some embodiments, the immunogenic composition comprises an immunogen plus an immunopotentiator or biological response modifier. The immunopotentiator or biological response modifier can be, for example, but is not limited to a cytokine, a chemokine a PAMP, a TLR-ligand, an immunostimulatory sequence, a CpG-containing DNA, a dsRNA, an endocytic-Pattern Recognition Receptor (PRR) ligand, an LPS, a quillaja saponin, a tucaresol, and the like.

The plurality of doses can be 2 or more doses. In some embodiments, the plurality of doses comprises 2 to 6 doses. In other embodiments, the plurality of doses comprises more than six doses. In some embodiments of the invention, the plurality of doses can be affected by the half-life (t_{1/2}) of the immunogen. For example, an immunogen with a relatively shorter half-life can require more frequent administration, and thus a greater number of doses, than an immunogen with a relatively longer half-life to achieve similar results.

In some embodiments, the last dose can be administered within 6 days of the first dose. In some embodiments, the last dose can be administered within 7, 8, 9, 10 or more days after the first dose.

Embodiments of the invention relate to methods wherein an enhanced response is obtained as compared to an immunization utilizing the same cumulative dose without a linear or an exponential increase in dosage over time. The enhanced response can comprise an increased number of responding T cells. In some embodiments, the enhanced response can comprise increased production of an immunostimulatory cytokine. The cytokine can be, for example, IL-2 or IFN-γ. In some embodiments, the enhanced response can comprise an increase in cytolytic activity. In some embodiments, the enhanced response can comprise a delay in peak production of an immunosuppressive cytokine. The immunosuppressive cytokine can be, for example, IL-10.

Embodiments of the invention relate to methods of administering an immunogenic composition to a mammal by delivery directly to the lymphatic system. For example, the method of administering an immunogenic composition to a mammal can be by intranodal delivery.

In some embodiments of the invention, the immunogenic composition can be administered to a mammal subcutaneously, intramuscularly, intradermally, transdermally, transmucosally, nasally, bronchially, orally, rectally or the like.

In some embodiments, the immunogen can be provided as a protein, peptide, polypeptide, naked DNA vaccine, RNA vaccine, synthetic epitope, mimotope, or the like, but is preferably not limited to such.

The immunogen stimulates a response to an antigen associated with the disease to be treated or protected against. The antigen can be, for example, but is not limited to, a viral antigen, a bacterial antigen, a fungal antigen, a differentiation antigen, a tumor antigen, embryonic antigen, an antigen of oncogenes and mutated tumor-suppressor genes, a unique tumor antigen resulting from chromosomal translocations, and the like and/or derivatives thereof. The antigen can be a self-antigen.

In some embodiments, the immunopotentiator can be a TLR-ligand. The TLR-ligand can be a CpG-containing DNA. In some embodiments, the immunopotentiator can be double-stranded RNA, for example poly IC.

Some embodiments of the invention disclosed herein relate to a set of immunogenic compositions, wherein the set includes an immunogen, plus an immunopotentiator or biological response modifier, wherein the dosages of the individual members of the set are related as an exponential series. In some embodiments, the exponential series of dosages are defined by an exponential factor ≥ 2ⁿ⁻¹. In some embodiments, the exponential series of dosages are defined by an exponential factor of 5ⁿ⁻¹.

Other embodiments of the invention relate to a kit comprising the set of immunogenic compositions comprising an antigen and an immunopotentiator or biological response modifier and instructions for administering the compositions to a subject in need thereof.

The immunopotentiator or biological response modifier can be, for example, but is not limited, a cytokine, a chemokine a PAMP, a TLR-ligand, an immunostimulatory sequence, a CpG-containing DNA, a dsRNA, an endocytic-Pattern Recognition Receptor (PRR) ligand, an LPS, a quillaja saponin, a tucaresol, and the like. In some embodiments, the immunogen, and the immunopotentiator or biological response modifier, can each be contained in separate containers or in the same container.

In some embodiments of the invention, the kit can comprise two or more doses of an immunogenic composition each in a separate suitable container. The suitable container can be, for example, but is not limited to, a syringe, an ampule, a vial, and the like, or a combination thereof.

Embodiments of the invention relate to a set of syringes comprising sequentially increasing doses of an immunogenic composition wherein each dose subsequent to an initial dose is greater than the immediately preceding dose in each syringe of the set of syringes and, wherein the immunogenic composition comprises an immunogen, and a immunopotentiator or biological response modifier, to enhance a T-cell response in a subject.

In other embodiments, the immunogenic composition comprises a cell. The cell can be a tumor cell or an antigen presenting cell, is but not limited to such. In other embodiments, the antigen presenting cell can be a dendritic cell. In still other embodiments, the immunogenic composition comprises a cell.

In additional embodiments, the greater dose comprises an increased number of cells. In yet another embodiment, the greater dose comprises an increased number of epitope-MHC complexes on the surface of the cell.

Some embodiments relate to a set of vials comprising sequentially increasing doses of an immunogenic composition wherein each dose subsequent to an initial dose is greater than the immediately preceding dose in each vial of the set of vials and, wherein the immunogenic composition comprises an immunogen, and a immunopotentiator or biological response modifier, to enhance a T-cell response in a subject

Some embodiments relate to the use of a plurality of sequential doses of an immunogenic composition for stimulating a class I MHC-restricted T cell response, wherein each dose subsequent to an initial dose is greater than the immediately preceding dose. In some embodiments, stimulating a class I MHC-restricted T cell response is for the treatment of a neoplastic disease or for the treatment of an infectious disease, or both. In some embodiments, stimulating a class I MHC-restricted T cell response is for the prevention of a neoplastic disease or for the prevention of an infectious disease, or both.

Some embodiments relate to the use of a plurality of sequential doses of an immunogenic composition comprising an immunogen, and an immunopotentiator or biological response modifier, in the manufacture of a medicament, wherein each dose subsequent to an initial dose is greater than the immediately preceding dose. Some embodiments relate to the use of a set of immunogenic compositions comprising an immunogen, plus an immunopotentiator or biological response modifier, in the manufacture of a medicament, wherein the dosages of the individual members of the set are related as an exponential series. Preferably, the medicament stimulates a class I MHC-restricted T cell response in a mammal. Thus, the medicament can be for the treatment of a neoplastic disease or for the treatment of an infectious disease, or both. In some embodiments, the medicament is for the prevention of a neoplastic disease or for the prevention of an infectious disease, or both.

Other objects and features will be in part apparent and in part pointed out hereinafter.

### BRIEF DESCRIPTION OF THE DRAWINGS

The following drawings form part of the present specification and are included to further demonstrate certain aspects of embodiments of the invention disclosed herein. The invention can be better understood by reference to one or more of these drawings in combination with the detailed description of specific embodiments presented herein.

FIGURE 1 illustrates data that indicate that exponentially increasing doses of both gp33 and CpG enhance CD8⁺ T cell response.

FIGURE 2 is a bar graph that indicates that enhancement of the CD8⁺ T cell response is independent of T cell help.

FIGURE 3 shows data indicating that four days of antigen stimulation is optimal for CD8⁺ T cell induction.

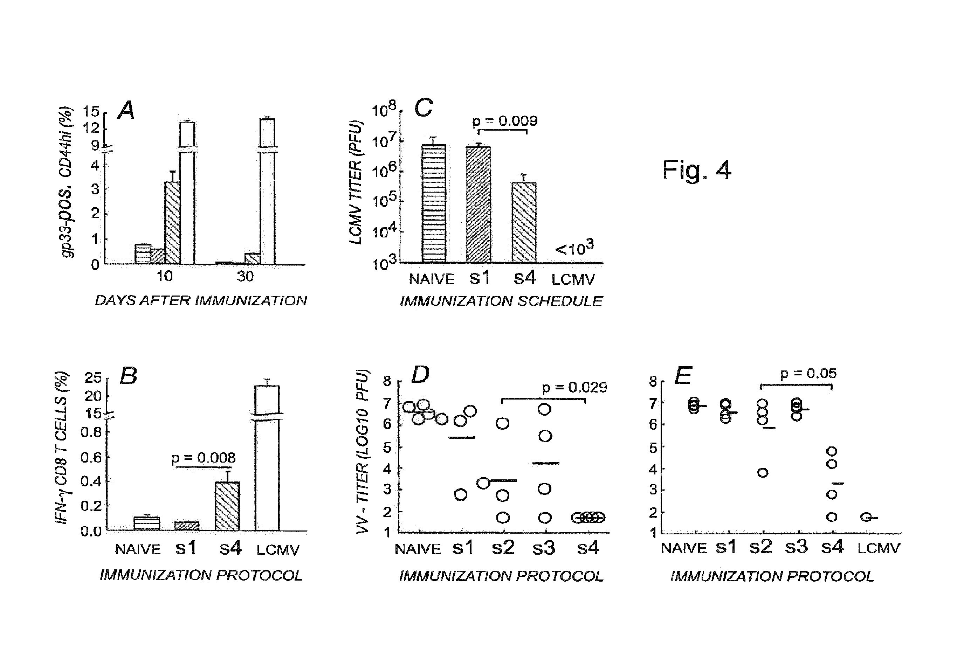

FIGURE 4 illustrates data that indicate that exponentially increasing doses of both gp33 and CpG enhance antiviral CD8⁺ T cell responses.

FIGURE 5 shows data that indicate that antigen kinetics does not affect DC activation.

FIGURE 6A illustrates flow cytometry data indicating that exponential immunization favors persisting T cell proliferation.

FIGURE 6B illustrates flow cytometry data indicating that exponential immunization favors persisting T cell proliferation.

FIGURE 7 shows data indicating that exponential immunization with peptide-loaded dendritic cells induces strong T cell and anti-tumor responses.

FIGURE 8 illustrates data that indicate that exponential in vitro stimulation of CD8⁺ T cells enhances IL-2 production and cytotoxicity.

### DETAILED DESCRIPTION OF THE INVENTION

The immune system has evolved to optimally respond to pathogens (Janeway, C.A., Jr. Approaching the asymptote? Evolution and revolution in immunology. Cold Spring Harb Symp Quant Biol 54 Pt 1, 1-13, 1989; Zinkernagel, R.M., Science 271, 173-8, 1996; Germain, R.N., Nat Med 10, 1307-20, 2004, each of which is incorporated herein by reference in its entirety). Immunization can be optimized, and efficacy of baccines can be enhanced, by adopting characteristics of pathogens. For example, to enhance phagocytosis and antigen presentation, vaccines can be delivered in a particulate form with comparable dimensions to pathogens, such as emulsions, microparticles, iscoms, liposomes, virosomes and virus like particles to enhance phagocytosis and antigen presentation (O'Hagan, D.T. & Valiante, N.M. Nat Rev Drug Discov 2, 727-35, 2003, which is incorporated herein by reference in its entirety). In addition, pathogen associated molecular patterns (PAMPs) stimulating the immune system via pattern recognition receptors (PRR), including toll-like receptors (TLR), can be used as adjuvants to activate antigen presenting cells and to enhance the immune response to vaccines (Johansen, P., et al., Clin Exp Allergy 35, 1591-1598, 2005b; O'Hagan, D.T. & Valiante, N.M. Nat Rev Drug Discov 2, 727-35, 2003; Krieg, A.M., Annu Rev Immunol 20, 709-60, 2002, each of which is incorporated herein by reference in its entirety). One key hallmark of pathogens is replication. Pathogen replication exposes the immune system to increasing amounts of antigen and immunostimulatory PAMPs over time.

A current paradigm in immunology is that the strength and quality of T cell responses can be governed by the dose and localization of antigen as well as by co-stimulatory signals. Strategies to improve the efficiency of vaccination can be aimed at increasing the duration of antigen presentation (Lofthouse, S. Adv Drug Deliv Rev 54, 863-70, 2002; Ehrenhofer, C. & Opdebeeck, J.P., Vet Parasitol 59, 263-73, 1995; Guery, J.C., et al., J Exp Med 183, 485-97,1996; Zhu, G., et al., Nat Biotechnol 18, 52-7, 2000; Borbulevych, O.Y., et al., J Immunol 174, 4812-20, 2005; Levitsky, V., et al., J Exp Med 183, 915-26, 1996; van der Burg, S.H., et al., J Immuno 156, 3308-14; 1996; Chen, J.L. et al., J Exp Med 201, 1243-55; 2005; Rivoltini, L. et al., Cancer Res 59, 301-6,1999; Blanchet, J.S. et al., J Immunol 167, 5852-61, 2001; Brinckerhoff, L.H. et al., Int J Cancer 83, 326-34; 1999; Ayyoub, M. et al., J Biol Chem 274, 10227-34, 1999; Stemmer, C. et al., J Biol Chem 274, 5550-6,1999; O'Hagan, D.T. & Valiante, N.M., Nat Rev Drug Discov 2, 727-35, 2003, each of which is incorporated herein by reference in its entirety).

Methods of optimizing T cell induction still remain a challenge in the art. The "depot" theory of immunization, as is well known in the art, postulates that antigen slowly leaking into the tissues over an extended time correlates with the immunogenic potency of a vaccine. Currently, this antigen depot paradigm serves as the backbone to most adjuvant development programs. However, the present disclosure demonstrates that it is far more advantageous to administer a vaccine in a dose escalating fashion over several consecutive or closely spaced days rather than in a depot formulation or as one single bolus. Daily antigenic stimulation with exponentially increasing doses is shown herein to enhance the CD8⁺ T cell response when compared to single bolus or multiple unchanging dose administrations. As used herein, stimulating a class I MHC-restricted T cell response includes without limitation inducing, priming, initiating, prolonging, maintaining, amplifying, augmenting, or boosting the response.

It is well known in the art that live attenuated vaccines usually induce strong and long lasting immune responses after one injection, and that many viral vaccines of this type have efficiencies greater than 90% (Nossal, G. Vaccines in Fundamental Immunology (ed., Paul, W.E.) 1387-1425; Lippincot-Raven Publishers, Philadelphia, 1999, which is herein incorporated by reference in its entirety). In contrast, vaccines consisting of killed microorganisms, toxins, subunit vaccines including peptide vaccines, or naked DNA vaccines, are of considerably lower efficacy, and boosting immunizations are essential. While live vaccines produce increasing antigen doses that call for strong immune responses, non-replicating vaccines produce a decreasing antigen profile that is, as demonstrated in the examples herein, a rather weak stimulus for T cells.

It is well-known that replication -incompetent vaccines are safer than live vaccines. Embodiments of the invention serve to challenge the trend in vaccine development to use replication-incompetent vaccines without regard to the dose-kinetics of antigenic stimulation. Further, embodiments of the invention provide an immunotherapeutic approach to enhance T cell responses against diseases such as, but not limited to, infectious diseases or cancer.

Embodiments of the invention disclosed herein are aimed at addressing the deficiencies in the art of vaccine design and in the practice of immunotherapy by manipulating the kinetics of antigenic stimulation as a key parameter of immunogenicity. As disclosed herein, immunogenic stimulation that was linearly or exponentially increased induced significantly stronger CD8⁺ T cell responses relative to stimulation that was provided at a constant level. Immunogens that were given as a single shot or as multiple decreasing doses induced the weakest immune responses. An evolutionary explanation for the findings disclosed herein can be that pathogens that replicate and therefore produce increasing amounts of antigen require the strongest CD8⁺ T cell response. In contrast, uniform or decreasing amounts of antigen indicate non-pathogenic stimuli or infections well controlled by innate or already ongoing acquired immunity.

While not wishing to be bound by this theory, it is believed that the likely candidates for mediating increased sensitivity to danger are antigen-presenting cells and, particularly for CD8⁺ T cell induction, dendritic cells (DCs). This is supported in the examples below in that while the different vaccination protocols did not differ in the absolute DC numbers or the level of DC activation in the lymph node, they differed in the time it took to reach the peak of DC activation and numbers. In the exponentially increasing vaccination model, the peak of DC activation was delayed three days as compared to bolus vaccination. With both vaccination regimes, the peak of DC activation occurred one day after the maximal vaccine dose was administered. This observation could imply that an optimal vaccination schedule would employ a single injection of CpG preceding a single injection of peptide by one day, such that the peptide is presented on maximally activated DCs. However, the presented data demonstrate that such an immunization protocol, as well as exponentially increasing doses of CpG followed by a single dose of peptide, was significantly less immunogenic than exponentially escalating doses of CpG and peptide in parallel.

To examine whether the dose-kinetics of antigen, independent of the total dose over the course of treatment (the cumulative dose), is a separate parameter of immunogenicity, mice were immunized with a fixed cumulative dose of an antigenic peptide, such as gp33, and an immunopotientiator or biological response modifier (BRM), such as cytosine-guanine oligodeoxynucleotides (CpG ODNs). Different kinetics, i.e., immunization with exponentially increasing or decreasing doses, constant daily doses, or single bolus immunization were implemented. MHC class-I binding peptides were chosen as antigens, since their short in vivo half-life allows for the production of sharp antigen kinetics (Falo et al., Proc Natl Acad Sci U S A 89, 8347-8350, 1992; Widmann et al., J Immunol. 147, 3745-3751, 1991, each of which is incorporated herein by reference in its entirety). Since mice were immunized with the same total dose of the vaccine, specific T cell induction could be monitored as a function of the kinetics of peptide and BRM (CpG) administration.

As disclosed in the examples and elsewhere, herein, fixed cumulative vaccine doses comprising both peptide and BRM (CpG) were administered by different schedules to produce distinct dose-kinetics of antigenic stimulation. Exponentially increasing antigenic stimulation mounted a significantly stronger stimulus for CD8⁺ T cells and enhanced long-term immunity against viral infections and tumors compared to uniform or constant daily antigenic stimulation or administration of the vaccine as a single bolus. The same phenomenon was observed when T cells were stimulated *in vitro.*

Thus, some embodiments relate to methods and compositions for linearly or exponentially increasing antigenic stimulation of class I MHC CD8⁺ T cell responses over that described in the art. The data shows increasing antigenic stimulation independent of the antigen dose enhanced immunogenicity. Therefore, the invention provides a novel method for enhancing immunogenicity, thereby improving vaccine development.

Embodiments of the invention provide sets of immunogenic compositions comprising an immunogen, plus an immunopotentiator or BRM. Some embodiments involve the co-administration of an antigen with an immunopotentiator to obtain an enhanced immune (CTL) response by providing both the antigen and the immunopotentiator in an exponentially increasing manner.

The immunogenicity of an antigen can be determined by a number of parameters including the antigen dose (Mitchison, N.A., Proc R Soc Lond Biol Sci 161, 275-92, 1964; Weigle, W.O., Adv Immunol 16, 61-122, 1973; Nossal, G.J., Annu Rev Immunol 1, 33-62, 1983, each of which is incorporated herein by reference in its entirety); the localization of the antigen (Zinkemagel, R.M., Semin Immunol 12, 163-71; discussion 257-344, 2000; Zinkemagel, R.M. & Hengartner, H., Science 293, 251-3, 2001, each of which is incorporated herein by reference in its entirety); the particulate or soluble nature of the antigen (O'Hagan, D.T. & Valiante, N.M. Nat Rev Drug Discov 2, 727-35, 2003; Bachmann, M.F., et al., Science 262, 1448-51,1993, each of which is incorporated herein by reference in its entirety); and whether or not the antigen is presented together with co-stimulatory signals (Janeway, C.A., Jr. Approaching the asymptote? Evolution and revolution in immunology. Cold Spring Harb Symp Quant Biol 54 Pt 1, 1-13, 1989; Germain, R.N., Nat Med 10, 1307-20, 2004; Matzinger, P., Annu Rev Immunol 12, 991-1045,1994; Schwartz, R.H., Cell 71, 1065-8, 1992, each of which is incorporated herein by reference in its entirety).

It is further thought that the more an immunogen and an immunization protocol resemble infection with a virulent pathogen, the more immunogenic it will be. High antigen doses and the presence of antigen in lymphoid organs, both corresponding to widespread replication of a virulent pathogen, induce strong immune responses. Particulate antigens that resemble the structure of viruses or bacteria induce stronger immune responses than soluble antigens. Additionally, presentation of an antigen together with pathogen components such as, for example, bacterial DNA, lipopolysaccharide or viral RNA strongly enhances the immune response. As taught herein, exponentially increasing antigenic stimulation can also be recognized by the immune system as a pattern associated with pathogens, driving strong immune responses.

Thus, in light of the aforementioned, an antigen contemplated for use in embodiments of the invention is one that stimulates the immune system of a subject having a malignant tumor or infectious disease to attack the tumor or pathogen to inhibit its growth or eliminate it, thereby treating or curing the disease. The antigen, in some instances, can be matched to the specific disease found in the animal being treated to induce a CTL response (also referred to as a cell-mediated immune response), i.e., a cytotoxic reaction by the immune system that results in lysis of the target cells (e.g., the malignant tumor cells or pathogen-infected cells). As understood by one of ordinary skill in the art, an increased cytolytic activity can be a measure of the number of target cells killed or lysed in the presence of the immunogenic composition relative to that in the absence of the immunogenic composition. Methods to determine or measure the number of target cells killed or lysed can be any method known to one of ordinary skill in the art including, but not limited to, a chromium release assay, a tetramer assay, and the like.

As used herein, stimulating a class I MHC-restricted T cell response includes without limitation inducing, priming, initiating, prolonging, maintaining, amplifying, augmenting, or boosting the response.

Antigens contemplated as useful in the methods disclosed herein include, but are not limited to proteins, peptides, polypeptides and derivatives thereof, as well as non-peptide macromolecules. Such a derivative can be prepared by any method known to those of ordinary skill in the art and can be assayed by any means known to those of ordinary skill in the art. Accordingly, in some embodiments, antigens for use in the present invention can include tumor antigens such as, but not limited to, differentiation antigens, embryonic antigens, cancer-testis antigens, antigens of oncogenes and mutated tumor-suppressor genes, unique tumor antigens resulting from chromosomal translocations, viral antigens, and others that can be apparent presently or in the future to one of skill in the art. Antigens useful in the disclosed methods and compositions also include those found in infectious disease organisms, such as structural and non-structural viral proteins. Potential target microbes contemplated for use in the disclosed compositions and methods, include without limitation, hepatitis viruses (*e.g*., B, C, and delta), herpes viruses, HIV, HTLV, HPV, EBV, and the like. A general term for these antigens, which are to be recognized or targeted by the immune response, is target-associated antigen (TAA).

Protein antigens that can be employed in the disclosed methods and compositions include, but are not limited to: differentiation antigens such as, for example, MART-1/MelanA (MART-I), gp100 (Pmel 17), tyrosinase, TRP-1, TRP-2 and tumor-specific multilineage antigens such as, for example, MAGE-1, MAGE-3, BAGE, GAGE-1, GAGE-2, p15; overexpressed embryonic antigens such as, for example, CEA; overexpressed oncogenes and mutated tumor-suppressor genes such as, for example, p53, Ras, HER-2/neu; unique tumor antigens resulting from chromosomal translocations such as, for example, BCR-ABL, E2A-PRL, H4-RET, IGH-IGK, MYL-RAR; and viral antigens, such as, for example, the Epstein Barr virus antigens EBVA and the human papillomavirus (HPV) antigens E6 and E7. Other protein antigens can include, for example: TSP-180, MAGE-4, MAGE-5, MAGE-6, RAGE, NY-ESO, p185erbB2, p180erbB-3, c-met, nm-23HI, PSA, TAG-72, CA 19-9, CA 72-4, CAM 17.1, NuMa, K-ras, β-Catenin, CDK4, Mum-1, p15, p16, 43-9F, 5T4, 791Tgp72, alpha-fetoprotein, β-HCG, BCA225, BTAA, CA 125, CA 15-3\CA 27.29\BCAA, CA 195, CA 242, CA-50, CAM43, CD68\KP1, CO-029, FGF-5, G250, Ga733\EpCAM, HTgp-175, M344, MA-50, MG7-Ag, MOV18, NB/70K, NY-CO-1, RCAS1, SDCCAG16, PLA2, TA-90\Mac-2 binding protein\cyclophilin C-associated protein, TAAL6, TAG72, TLP, and TPS. These protein-based antigens are known and available to the skilled artisan both in the literature or commercially.

In other instances of the invention, peptide antigens of 8-15 amino acids in length are contemplated. Such a peptide can be an epitope of a larger antigen, i.e., it is a peptide having an amino acid sequence corresponding to the site on the larger molecule that is presented by MHC/HLA molecules and can be recognized by, for example, an antigen receptor or T cell receptor. These smaller peptides are available to one of skill in the art and can be obtained, for example, by following the teachings of U.S. Patent Nos. 5,747,269 and 5,698,396; and PCT Application Number PCT/EP95/02593 (published as WO 96/01429), filed July 4, 1995, entitled METHOD OF IDENTIFYING AND PRODUCING ANTIGEN PEPTIDES AND USE THEREOF AS VACCINES and PCT Application No. PCT/DE96/00351 (published as WO 96/27008), filed February 26, 1996, entitled AGENT FOR TREATING TUMOURS AND OTHER HYPERPLASIA, each of which is incorporated herein by reference in its entirety. Additional approaches to epitope discovery are described in U.S. Patent Nos. 6,037,135 and 6,861,234, each of which are incorporated herein by reference in its entirety.

While in general the molecule ultimately determining antigen-specific recognition by a T cell is a peptide, it is noted that the form of antigen actually administered in the immunogenic preparation, the immunogen, need not be a peptide per se. When administered, the epitopic peptide(s) can reside within a longer polypeptide, whether as the complete protein antigen, some segment of it, or some engineered sequence. Included in such engineered sequences would be polyepitopes and epitopes incorporated into a carrier sequence such as an antibody or viral capsid protein. Such longer polypeptides can include epitope clusters as described, for example, in U.S. Patent Application No. 09/561,571, filed April 28, 2000 and entitled "EPITOPE CLUSTERS," which is incorporated herein by reference in its entirety. The epitopic peptide, or the longer polypeptide in which it is contained, can be a component of a microorganism (*e.g.,* a virus, bacterium, protozoan, *etc*.), or a mammalian cell (*e.g.,* a tumor cell or antigen presenting cell), or lysates, whole or partially purified, of any of the foregoing. They can be used as complexes with other proteins, for example heat shock proteins. The epitopic peptide can also be covalently modified, such as by lipidation, or made a component of a synthetic compound, such as dendrimers, multiple antigen peptides systems (MAPS), and polyoximes, or can be incorporated into liposomes or microspheres, and the like. As used in this disclosure the term "polypeptide antigen" encompasses all such possibilities and combinations. The invention comprehends that the antigen can be a native component of the microorganism or mammalian cell. The antigen can also be expressed by the microorganism or mammalian cell through recombinant DNA technology or, especially in the case of antigen presenting cells, by pulsing the cell with polypeptide antigen or epitopic peptide prior to administration. Additionally, the antigen can be administered encoded by a nucleic acid that is subsequently expressed by APCs. Finally, whereas the classical class I MHC molecules present peptide antigens, there are additional class I molecules which are adapted to present non-peptide macromolecules, particularly components of microbial cell walls, including without limitation lipids and glycolipids. As used in this disclosure the terms antigen, immunogen, and epitope can include such macromolecules as well. Moreover, a nucleic acid based vaccine can encode an enzyme or enzymes necessary to the synthesis of such a macromolecule and thereby confer antigen expression on an APC.

It is also contemplated that new peptides identified by the method disclosed in U.S. Patent Application Serial No. 09/560,465, filed April 28, 2000 and entitled "EPITOPE SYNCHRONIZATION IN ANTIGEN PRESENTING CELLS," (incorporated herein by reference in its entirety) that can be apparent presently or in the future to one of ordinary skill in the art, are useful in embodiments of the invention disclosed herein.

Additional peptides, and peptide analogues that can be employed in embodiments of the invention disclosed herein are disclosed, for example, in U.S. Provisional Application No. 60/581,001, filed on June 17, 2004 and U.S. Patent Application No. 11/156,253, filed June 17, 2005, both entitled SSX-2 PEPTIDE ANALOGS; and U.S. Provisional Application No. 60/580,962, filed June 17, 2004 and U.S. Patent Application No. 11/155,929, filed June 17, 2005, both entitled NY-ESO PEPTIDE ANALOGS; U.S. Patent Application No. 09/999,186, filed November 7, 2001, entitled METHODS OF COMMERCIALIZING AN ANTIGEN; U.S. Patent Application No. 11/323,572 (published as US 2006/0165711 A1), filed on December 29, 2005, entitled, METHODS TO ELICIT, ENHANCE AND SUSTAIN IMMUNE RESPONSES AGAINST MHC CLASS I- RESTRICTED EPITOPES, FOR PROPHYLACTIC OR THERAPEUTIC PURPOSES; and U.S. Patent Application No. 11/323,520, filed on December 29, 2005, entitled METHODS TO BYPASS CD4⁺ CELLS IN THE INDUCTION OF AN IMMUNE RESPONSE, each of which is incorporated herein by reference in its entirety. Beneficial epitope selection principles for immunotherapeutics are disclosed, for example, in U.S. Patent Application No. 09/560,465, filed on April 28, 2000, U.S. Patent Application No. 10/026,066 (published as US 2003/0215425 A1), filed on December 7, 2001, and U.S. Patent Application No. 10/005,905, filed on November 7, 2001, all entitled EPITOPE SYNCHRONIZATION IN ANTIGEN PRESENTING CELLS; U.S. Patent Application No. 09/561,571, filed on April 28, 2000 and entitled EPITOPE CLUSTERS; U.S. Patent Application No. 10/094,699 (now U.S. Patent 7,252,824), filed on March 7, 2002 and entitled ANTI-NEOVASCULATURE PREPARATIONS FOR CANCER; U.S. Patent Application No. 10/117,937 (published as US 2003/0220239 A1), filed on April 4, 2002, U.S. Patent Application No. 10/657,022 (published as US 2004/0180354 A1), filed on September 5, 2003, and PCT Application No. PCT/US2003/027706 (published as WO 04/022709A2), filed September 5, 2003, all entitled EPITOPE SEQUENCES; and U.S. Patent No. 6,861,234; each of which is incorporated herein by reference in its entirety.

In some aspects of the invention, vaccine plasmids can be used. The overall design of vaccine plasmids are disclosed, for example, in U.S. Patent Application No. 09/561,572, filed on April 28, 2000 and entitled EXPRESSION VECTORS ENCODING EPITOPES OF TARGET-ASSOCIATED ANTIGENS; U.S. Patent Application No. 10/292,413 (published as US 2003/0228634 A1), filed on November 7, 2002 and entitled EXPRESSION VECTORS ENCODING EPITOPES OF TARGET-ASSOCIATED ANTIGENS AND METHODS FOR THEIR DESIGN; U.S. Patent Application No. 10/225,568 (published as US 2003/0138808), filed on August 20, 2002 and PCT Application No. PCT/US2003/026231 (published as Publication No. WO 2004/018666), filed August 19, 2003, both entitled EXPRESSION VECTORS ENCODING EPITOPES OF TARGET-ASSOCIATED ANTIGENS; and U.S. Patent No. 6,709,844, entitled "AVOIDANCE OF UNDESIRABLE REPLICATION INTERMEDIATES IN PLASMIND PROPAGATION", each of which is incorporated herein by reference in its entirety.

Further contemplated in embodiments of the invention are specific antigenic combinations of particular benefit in directing an immune response against particular cancers as disclosed, for example, in U.S. Provisional No. 60/479,554, filed on June 17, 2003, and U.S. Patent Application No. 10/871,708 (published as US 2005/0118186), filed on June 17, 2004, U.S. Patent Application No. 11/323,049 (published as US 2006/0159694 A1), filed December 29, 2005, and PCT Patent Application No. PCT/US2004/019571, filed June 17, 2004, all entitled "COMBINATIONS OF TUMOR-ASSOCIATED ANTIGENS IN VACCINES FOR VARIOUS TYPES OF CANCERS", each of which is incorporated herein by reference in its entirety.

An epitope as referred herein and as is well known to the skilled artisan, is defined as that portion of an antigen that interacts with an antigen receptor of an immune system; in the present case that portion of an antigen presented by an MHC molecule for recognition by a T cell receptor (TCR). An immunogen is a molecule capable of stimulating an immune response. Immunogens as contemplated in invention disclosed herein can include, in a nonlimiting manner, a polypeptide or a nucleic acid encoding a polypeptide, wherein the polypeptide is capable of stimulating an immune response. Immunogens can be identical to a corresponding TAA or a fragment of thereof, but are not necessarily so. Immunogens can include, but are not necessarily limited to, epitopic peptides presented on the surface of cells and peptides non-covalently bound (complexed) to the binding cleft of class I MHC, such that they can interact with T cell receptors (TCR). Additionally immunogens can include epitope-MHC complexes or cells expressing such complexes on their surface.

A mimotope, as referred herein and as is well known to the skilled artisan, is defined as a compound that mimics the structure of an epitope and provokes an identical or cross-reactive immune response. A synthetic epitope, as referred to herein and as is well known to the skilled artisan, is a chemically synthesized non-natural epitope molecule. Methods for synthesizing proteins, peptides and the like are well known in the art.

### Immunopotentiators and Biological Response Modifiers (BRMs)

Embodiments of the invention disclosed herein include methods of enhancing a T cell immune response by administration of an immunogenic composition comprising an immunogen plus an immunopotentiator or other biological response modifier (BRM). BRMs can act in an immunosuppressive or immunostimulatory manner to modulate an immune response, for example, by promoting an effector response or inhibiting a T regulatory response. Immunopotentiators or BRMs as used herein can refer to any molecule that modulates the activity of the immune system, or the cells thereof, through an interaction other than with an antigen receptor. BRMs as used herein can further include natural or synthetic small organic molecules which exert immune modulating effects by stimulating pathways of innate immunity.

Preferred immunpotentiating BRMs that can be utilized in embodiments of the invention are molecules that trigger cytokine or chemokine production, such as, but not limited to, ligands for Toll-like receptors (TLRs), peptidoglycans, LPS or analogues, imiquimodes, unmethylated CpG oligodeoxynuclotides (CpG ODNs), dsRNAs, such as bacterial dsDNA (which contains CpG motifs) and synthetic dsRNA (polyI:C) on APC and innate immune cells that bind to TLR9 and TLR3, respectively, and the like. It is noted that these BRMs are potent immune modulators associated with safety concerns when delivered systemically. CpG especially (TLR-9 ligand) has shown widespread experimental application and clinical potential as an adjuvant by allowing efficient maturation of antigen-presenting cells and subsequent activation of antigen-specific lymphocytes (Krieg, A. M., Annu Rev Immunol 20: 709-760 2002; Weigel, B. J. et al., Clin. Cancer Res. 9: 3105-3114, 2003; Verthelyi, D. et al., Aids 18: 1003-1008, 2004; Storni, T. et al., J Immunol 172: 1777-1785, 2004; each of which is incorporated herein by reference in its entirety). One approach to avoiding these safety concerns is the use of intralymphatic administration as disclosed, for example, in U.S. Patent Application No. 11/321,967 (published as US. 2006/0153844 A1), filed December 29, 2005 and entitled METHODS TO TRIGGER, MAINTAIN, AND MANIPULATE IMMUNE RESPONSES BY TARGETED ADMINISTRATION OF BIOLOGICAL RESPONSE MODIFIERS INTO LYMPHOID ORGANS, which is incorporated herein by reference in its entirety.

As used herein, the term BRM can refer to any molecule that modulates the activity of the immune system, or the cells thereof, through an interaction other than with an antigen receptor. BRM is also commonly applied to complex biological preparations comprising the active entity, or entities, without regard for whether the active component(s) of the mixture had been defined. Examples of complex biological preparations used as BRMs include OK 432, PSK, AIL, lentinan, and the like. In some embodiments of the invention the active component(s) of such a mixture are defined. In other embodiments of the invention BRMs sourced from complex biological preparations are at least partially purified, or substantially purified, such as, for example, OK-PSA (Okamoto et al., Journal of the National Cancer Institute, 95:316-326, 2003, which is incorporated herein by reference in its entirety) or AILb-A (Okamoto et al., Clinical and Diagnostic Laboratory Immunology, 11:483-495, 2004 which is incorporated herein by reference in its entirety). In preferred embodiments the BRM is of defined molecular composition. BRMs include immunopotentiating adjuvants that activate pAPC or T cells including, for example: TLR ligands, endocytic-Pattern Recognition Receptor (PRR) ligands, quillaja saponins, tucaresol, cytokines, and the like. Some preferred adjuvants are disclosed, for example, in Marciani, D.J. Drug Discovery Today 8:934-943, 2003, which is incorporated herein by reference in its entirety.

In some embodiments involving administration of cells as the immunogen, the BRM can be a molecule expressed by the cell. In one aspect, the BRM molecule can be expressed naturally by the cell either constitutively or in response to some biologic stimulus. In another aspect, expression depends on recombinant DNA or other genetic engineering technology.

One class of BRM includes mostly small organic natural or synthetic molecules, which exert immune modulating effects by stimulating pathways of innate immunity. It has been shown that macrophages, dendritic and other cells carry so-called Toll-like receptors (TLRs), which recognize pathogen-associated molecular patterns (PAMPs) on micro-organisms (Thoma-Uszynski, S. et al., Science 291:1544-1547, 2001; Akira, S., Curr. Opin. Immunol., 15: 5-11, 2003; each of which is incorporated herein by reference in its entirety). Further contemplated are small molecules that bind to TLRs such as a new generation of purely synthetic anti-viral imidazoquinolines, e.g., imiquimod and resiquimod, which have been found to stimulate the cellular path of immunity by binding the TLRs 7 and 8 (Hemmi, H. et al., Nat Immunol 3: 196-200, 2002; Dummer, R. et al., Dermatology 207: 116-118, 2003; each of which is incorporated herein by reference in its entirety).

BRMs that interact directly with receptors that detect microbial components are used in preferred embodiments. However, molecules that act downstream in the signalling pathway can also be used. Thus, antibodies that bind to co-stimulatory molecules (such as, for example, anti-CD40, CTLA-4, anti-OX40, and the like) can be used as BRMs in embodiments of the invention. Similarly, in still further embodiments, BRMs employed in embodiments of the invention can include, for example, IL-2, IL-4, TGF-beta, IL-10, IFN-gamma, and the like; or molecules that trigger their production. Still, other BRMs as contemplated herein by the present invention can include cytokines such as, for example, IL-12, IL-18, GM-CSF, flt3 ligand (flt3L), interferons, TNF-alpha, and the like; or chemokines such as IL-8, MIP-3alpha, MIP-lalpha, MCP-1, MCP-3, RANTES, and the like.

Adjuvants are molecules and preparations that improve the immunogenicity of antigens. They can have immunopotentiating activity as described above, but can also have, instead of or in addition to such activity, properties to alter the physical state of the immunogen. The effects of adjuvants are not antigen-specific. If they are administered together with a purified antigen, however, they can be used to selectively promote the response to the antigen. For example, the immune response is increased when protein antigens are precipitated by alum. Emulsification of antigens also prolongs the duration of antigen presentation. Suitable adjuvants include all acceptable immunostimulatory compounds, such as cytokines, toxins or synthetic compositions. Exemplary, often preferred adjuvants include, but are not limited, complete Freund's adjuvant (a non-specific stimulator of the immune response containing killed Mycobacterium tuberculosis), incomplete Freund's adjuvants, and aluminum hydroxide adjuvant.

One current procedure to render peptides more immunogenic is to inject them in the context of professional antigen presenting cells (APCs) such as dendritic cells (DCs), (Steinmann, R. M., Ann Rev Immunol 9, 271-96, 1991, which is incorporated herein by reference in its entirety). DCs are potent APCs of the immune system. Other adjuvants that can also be used include MDP compounds, such as, for example, thur-MDP and nor-MDP, CGP (MTP-PE), lipid A, and monophosphoryl lipid A (MPL). RIBI, which contains three components extracted from bacteria, MPL, trehalose dimycolate (TDM) and cell wall skeleton (CWS) in a 2% squalene/Tween 80 emulsion is also contemplated. Amphipathic and surface active agents, e.g., saponin and derivatives such as QS21 (Cambridge Biotech), form yet another group of adjuvants contemplated for use in embodiments of the present invention. Nonionic block copolymer surfactants (Rabinovich *et al.,* 1994, which is incorporated herein by reference in its entirety) can also be employed.

### Administration of Immunogenic Compositions of the Present Invention

Embodiments of the invention disclosed herein relate to methods of administering an immunogenic composition comprising an immunogen, plus an immunopotentiator or BRM, to a subject thereby inducing or enhancing an antigen-specific T cell response. In preferred embodiments, the immunogenic composition is provided to the subject in an exponentially increasing manner. In further embodiments, the immunogenic composition is provided to the subject in a linearly increasing manner.

According to the methods disclosed herein, the immunogenic composition can be delivered to a subject by any method known to one of ordinary skill in the art for delivering a composition. Thus, administration of an immunogenic composition of the present invention to a subject can be intradermally, intraperitoneally, intramuscularly, mucosally, and intranodally to the lymphoid organs (*e.g*., lymph nodes), but is not limited to such. For example, in some embodiments, administration of the immunogenic composition can be through transdermal, transmucosal, nasal, bronchial, oral, rectal and/or subcutaneous means. In some embodiments, administration of the immunogenic composition can comprise direct delivery to the lymphatic system. In other embodiments, administration of the immunogenic composition can consist of direct delivery to the lymphatic system. The human lymphatic system, as is well known to one of ordinary skill in the art, includes lymph, lymphocytes, lymph vessels, lymph nodes, tonsils, the spleen, the thymus gland, and bone marrow.

In some embodiments, it is desirable that an effective amount of the immunogenic composition comprising an immunogen, plus an immunopotentiator or a BRM be administered or delivered intranodally to a subject thereby eliciting an enhanced T cell response. In some embodiments, an enhanced response includes a linearly increased stimulation of a T cell response. In some embodiments, an enhanced response includes an exponentially increased stimulation of a T cell response. Intranodal administration is disclosed, for example, in U.S. Patent Nos. 6,994,851 and 6,977,074; PCT Patent Publication No. WO/9902183A2; and in U.S. Patent Publication Application No. 20050079152, each of which is incorporated herein by reference in its entirety. The integration of diagnostic techniques to assess and monitor immune responsiveness with methods of immunization is discussed more fully, for example, in U.S. Patent Application No. 11/155,928, filed June 17, 2005 and entitled "IMPROVED EFFICACY OF ACTIVE IMMUNOTHERAPY BY INTEGRATING DIAGNOSTIC WITH THERAPEUTIC METHODS", which is incorporated herein by reference in its entirety.

### Devices for Administration

The immunogenic compositions disclosed herein can be delivered by bolus injection with a hypodermic syringe, as in the examples below, or other similarly functional devices known in the art for vaccination. Other methods of delivery/administration can include infusion, for example subcutaneously or directly into the lymphatic system by an immunogen delivery vehicle, such as, for example, a pump. In preferred embodiments the delivery vehicle is external to the animal but contains a means (*e.g*., a needle or catheter) to deliver the antigen into the body, preferably to a lymphatic organ or area of high lymphatic flow. An advantage of an immunogen delivery vehicle is that it obviates multiple ongoing injections.

Delivery devices/vehicles positioned outside the patient's body (an external device), are comprised of a reservoir for holding the immunogenic composition, a programmable pump to pump the composition out of the reservoir, a transmission channel or line for transmitting the composition, and a means to introduce the composition into the patient's body to ultimately reach the lymphatic system. In a preferred embodiment, the pump can be programmed to ramp up the volume infused so as to provide the desired increasing immunogen concentration. In alternative embodiments, the pump's reservoir is filled with compositions comprising successively greater concentrations of immunogen. Preferably the reservoir for the immunogenic composition is large enough for delivery of the desired amount of immunogen over time and is easily refillable or replaceable without requiring the user to reinsert the means for introducing the immunogen composition to the lymph system. Use of external pumps for immunization, including exemplary pumps, is further discussed, for example, in U.S. Patent No. 6,997,074 entitled "*Method of Inducing a CTL Response*," which is incorporated herein by reference in its entirety.

### Treatment and Dosage Regimen

In general, embodiments the present invention are useful for treating a subject having a disease to which the subject's immune system mounts a cell-mediated response to a disease-related antigen in order to attack the disease. The type of disease can be, for example, a malignant tumor or an infectious disease caused by a bacterium, virus, protozoan, helminth, or any microbial pathogen that enters intracellularly and is attacked, e.g., by cytotoxic T lymphocytes. In a therapeutic modality the method is well-suited to persistent or chronic conditions, but is not necessarily limited to such. In addition, the present invention is useful for immunizing a subject that can be at risk of developing an infectious disease or tumor.

In treating diseases and/or conditions contemplated by the present invention, a dosage regimen and schedule of administration of the immunogenic composition comprising an immunogen plus an immunopotentiator or BRM can be employed. In some embodiments, the immunogenic composition disclosed herein can be administered as a plurality of sequential doses wherein each dose subsequent to an initial dose is an increased dose. Such a sequentially increasing dose can be provided as a linearly or exponentially increasing dose. As used herein, linearly increasing doses refers to a series of doses equal to ndᵢ where dᵢ is the initial dose and n is the index of the series, such that the dose series is dᵢ, 2dᵢ, 3dᵢ,...ndᵢ. By exponentially increasing doses, a series of doses equal to xⁿ⁻¹dᵢ, wherein x>1, such that the dose series is dᵢ, xdᵢ, x²dᵢ, x³dᵢ,...xⁿ⁻¹ dᵢ, is meant. Thus, if x=2 each dose is twice the immediately preceding dose in the series; if x=5 each dose is five times the immediately preceding dose in the series. Thus, in a preferred embodiment, the immunogenic composition of the invention can be administered as a plurality of sequential doses wherein each dose is provided at an exponential factor xⁿ⁻¹ times the initial dose. Such plurality of doses can be 2, 3, 4, 5, 6 or more doses as is needed. In instances where the initial dose(s) administered can be at too low a dose to generate an immune response, a greater number of doses (i.e., 7, 8, 9, 10, 12, 15 or more doses) can be administered to the subject to achieve a more immunologically effective dose response.

In some embodiments of the invention, the immunogenic composition comprises a cell comprising the antigen or an immunogenic portion thereof. In some of these embodiments the cell serves as an antigen presenting cell, either expressing and processing the antigen, or being pulsed with the antigen or an epitopic peptide or other immunogenic portion of the antigen. The cell may naturally express the antigen (or immunogen), for example a cancer cell expressing a TuAA, or may be manipulated to do so, for example a dendritic cell transfected with an mRNA encoding an immunogen. For example, the cell can be a cancer or tumor cell, or an antigen presenting cell, is but not limited to such. The tumor cell can be a bladder cell, a breast cell, a lung cell, a colon cell, a prostate cell, a liver cell, a pancreatic cell, a stomach cell, a testicular cell, a brain cell, an ovarian cell, a lymphatic cell, a skin cell, a brain cell, a bone cell, a soft tissue cell, or the like. The antigen presenting cell can be, for example, a dendritic cell. The dosage in these embodiments can be increased by sequentially increasing the number of cells administered relative to that of the immediately preceding dose, or by sequentially increasing the number of epitope-MHC complexes on the surface of the cells relative to that of the immediately preceding dose, wherein the epitope is from a target antigen, or both. The number of epitope-MHC complexes on the surface of the cells can be most readily manipulated by pulsing with different concentrations of the epitope.

Therefore, administration is in any manner compatible with the dosage formulation and in such amount as will be therapeutically or prophylactically effective. An effective amount or dose of an immunogenic composition of the invention is that amount needed to provide a desired response in the subject to be treated including, but not limited to: prevention, diminution, reversal, stabilization, or other amelioration of a disease or condition, its progression, or the symptoms thereof. The dosage of the immunogenic composition and dosage schedule can vary on a subject by subject basis, taking into account, for example, factors such as the weight and age of the subject, the type of disease and/or condition being treated, the severity of the disease or condition, previous or concurrent therapeutic interventions, the capacity of the individual's immune system to respond, the degree of protection desired, the manner of administration and the like, all of which can be readily determined by the practitioner.

The compositions of used herein can include various "unit doses." Unit dose is defined as containing a predetermined-quantity of the therapeutic composition calculated to produce the desired responses in association with its administration, i.e., the appropriate route and treatment regimen. The quantity to be administered, and the particular route and formulation, are within the skill of those in the clinical arts. Also of importance is the subject to be treated, in particular, the state of the subject and the protection desired. A unit dose need not be administered as a single injection but can comprise continuous infusion over a set period of time.

In further embodiments of the invention, it is contemplated that the plurality of doses of the immunogenic composition are administered within about 24 to 48 hrs of each other, within about 12-24 hr of each other, and most preferably within about 6-12 hr of each other, with an interval of about 24 hr between doses being most preferred. In some embodiments, it can be desirable to administer the plurality of doses of the immunogenic composition of the invention at an interval of days, where several days (for example, 1, 2, 3, 4, 5, 6 or 7) lapse between subsequent administrations. For example, the initial doses can be administered at a low dosage followed by a subsequent second low dose or high dose and such second dose can be administered at 1, 2, 3 or more days after the initial dose; a third dose can then be administered at 1, 2, 3 or more days after the second dose; a fourth dose can then be administered at 1, 2, 3 or more days after the third dose and so forth. In some embodiments, the sequential doses can be provided at an interval affected by the half-life of the antigen. The half-life of the antigen is the time it takes for fifty percent of the antigen to be metabolized or eliminated by normal biological processes from the subject. Thus, the skilled practitioner or clinical would determine the time period in which to administer the plurality of doses of the immunogenic composition and the time lapse between subsequent administrations in order to optimize a CD8⁺ T cell immune response.

The interval(s) of administration of an immunogenic composition of the invention can range from minutes to days depending on the dosage regimen and effectiveness of the dose administered. However, it is intended that the last dose be administered within a certain number of days of the first dose to enhance the number of responding T cells that correspond to the linear or exponential increase in the dose over time. In various embodiments, the time interval between the first and last dose can be less than 7 days, preferably it can be 4 or 5 days, and more preferably, the last dose can be administered within 6 days of the first dose. Thus, the last dose to be administered will not only depend on the day administered and the effectiveness of the initial doses, but will also be determined by the enhancement of the number of T cell to generate an immune response. Over time, the immune response elicited will decay and the procedure can be repeated to prolong or re-establish immunity.

A subject to which the immunogenic composition of the invention can be administered as a therapeutic can include humans of all ages and animals, such as, but not limited to, cattle, sheep, pigs, goats, and household pets such as dogs, cats, rabbits, hamsters, mice, rats, and the like. The immunogenic composition of the invention can primarily be utilized in treating humans that are in need of having a specific immunological response induced, sustained, or exponentially stimulated in the treatment of a disease or condition such as cancer or infectious disease.

### Kits

Any of the compositions described herein can be assembled together in a kit. In a non-limiting example, one or more agents or reagents for delivering an immunogenic composition can be provided in a kit alone, or in combination with an additional agent for treating a disease or condition due to infectious disease or cancer. However, these components are not meant to be limiting. The kits will provide suitable container means for storing and dispensing the agents or reagents.

Kits will generally contain, in suitable container means, an immunogenic composition comprising a pharmaceutically acceptable formulation of an immunogen, plus an immunopotentiator or BRM, for administering to a subject and instructions for administering. The kit can have a single container means, and/or it can have distinct container means for additional compounds such as an immunological/therapeutic effective formulation of a therapeutic agent(s) for treating a disease or condition due to infectious disease or cancer. The kit can further contain, in suitable container means, several doses of the immunogenic composition each in a separate container means. The several doses of immunogenic composition can be two or more sequentially increasing doses of an immunogenic composition, wherein each subsequent dose is greater than the dose immediately preceding it. In some embodiments, the kit contains two or more doses of an immunogenic composition, each dose in suitable separate container means. For example, the kit can contain 2, 3, 4, 5, 6, 7 or more doses of the immunogenic composition, each dose in suitable separate container means. In other embodiments, the kit can include several doses of the immunogen, or the immunopotentiator or BRM, each in separate container means.

Where the components of the kit are provided in one and/or more liquid solutions, the liquid solution is an aqueous solution, with a sterile aqueous solution being particularly preferred. The compositions can also be formulated into a syringeable composition, in which case, the container means can itself be a syringe, pipette, and/or other such like apparatus, from which the formulation can be delivered or injected into a subject, and/or even applied to and/or mixed with the other components of the kit. In some embodiments, the components of the kit can be provided as dried powder(s). When components (e.g., reagents) are provided as a dry powder, the powder can be reconstituted by the addition of a suitable solvent. It is envisioned that the solvent can also be provided in another container means.

The increasing dosages used according to the invention can be provided by administering ever larger volumes of the same concentration, or the same volume of ever greater concentration, or some combination thereof. Thus, in various embodiments kits can contain individually packaged doses; or one or more multidose containers from which increasing volumes are dispensed and administered; or one or more multidose containers from which a volume is dispensed, subjected to successively lesser dilution and a fixed volume administered; or similar assemblages and utilizations as will suggest themselves to one of skill in the art.

The container means will generally include at least one vial, ampule, test tube, flask, bottle, syringe and/or other container means, containing the immunogen and/or immunopotentiator or BRM. The kit can also comprise a second container means for containing a sterile, pharmaceutically acceptable buffer and/or other diluent. The kit of the present invention also will typically include a means for containing the materials for practicing the methods of the invention, and any other reagent containers in close confinement for commercial sale. Such containers can include injection or blow-molded plastic containers into which the desired vials are retained. Irrespective of the number or type of containers, the kit(s) of the invention can also comprise, or be packaged with, an instrument for assisting with the injection/administration of the immunogenic composition comprising an immunogen, plus an immunopotentiator or BRM within the body of a subject. Such an instrument can be a syringe, pump and/or any such medically approved delivery vehicle.

In some embodiments, a set of syringes containing increasing doses of the immunogenic composition is provided, wherein each dose subsequent to an initial dose is greater than the immediately preceding dose. In some embodiments, a set of vials containing increasing doses of the immunogenic composition is provided, wherein each dose subsequent to an initial dose is greater than the immediately preceding dose. The increasing doses can be sequentially increased by linear means or by exponential means.

Having described the invention in detail, it will be apparent that modifications, variations, and equivalent embodiments are possible without departing the scope of the invention defined in the appended claims. Furthermore, it should be appreciated that all examples in the present disclosure are provided as non-limiting examples.

### EXAMPLES

The following non-limiting examples are provided to further illustrate the present invention. It should be appreciated by those of skill in the art that the techniques disclosed in the examples that follow represent approaches the inventors have found function well in the practice of the invention, and thus can be considered to constitute examples of modes for its practice. However, those of skill in the art should, in light of the present disclosure, appreciate that many changes can be made in the specific embodiments that are disclosed and still obtain a like or similar result without departing from the spirit and scope of the invention.

### EXAMPLE 1: MATERIALS AND METHODS

**Mice**. Six to 12 weeks old C57BL/6 mice were purchased from Harlan (Horst, The Netherlands). TCR318 transgenic mice expressing a T cell receptor specific for peptide gp33 (aa33-41), which represents the immunodominant epitope of lymphocytic choriomeningitis virus (LCMV) in H-2^{b} mice, located in the glycoprotein (Pircher, H., et al. 1989. Nature 342:559-561; Pircher, H. et al., Nature 346, 629-633,1990, each of which is incorporated herein by reference in its entirety), were obtained from Cytos Biotechnology AG (Schlieren, Switzerland). HHD transgenic mice expressing HLA A2.1 were originally obtained from MannKind Corporation (Valencia, CA; Pascolo, S. et al., J Exp Med 185, 2043-2051, 1997, which is incorporated herein by reference in its entirety). Mice were bred and kept in a specific pathogen-free facility at the University Hospital of Zurich according to guidelines of the Swiss veterinary authorities.

**Viruses, peptides and oligodeoxynucleotides**. LCMV isolate WE was obtained from the Institute of Experimental Immunology, University Hospital, Zurich, Switzerland. LCMV titers were determined using a focus-forming assay on MC57 fibroblasts (Battegay, M. et al., J Virol Methods 33, 191-198; 1991, which is incorporated herein by reference in its entirety). Recombinant vaccinia virus expressing the LCMV glycoprotein (vacc-gp) (Bachmann, M.F. et al., Eur J Immunol 24, 2228-2236, 1994, which is incorporated herein by reference in its entirety) was grown and plaqued on BSC40 cells (Kundig, T.M. et al., J Virol 67, 3680-3683; 1993, which is incorporated herein by reference in its entirety). LCMV glycoprotein peptides gp33 (aa33-41; KAVYNFATM, SEQ ID NO:3) and gp61 (aa61-80; GLNGPDIYKGVYQFKSVEFD, SEQ ID NO:4) and VSV peptide np52 (SDLRGYVYQGLKSG, SEQ ID NO:5) were purchased from EMC Microcollections (Tübingen, Germany). Influenza matrix peptide (GILGFVFTL, SEQ ID NO:6) was obtained from Neosystems (Strasbourg, France). The HPV16 E7 (aa49-57; RAHYNIVTF, SEQ ID NO:7) peptide used was synthesized at MannKind Corporation (Valencia, CA) to >99% purity. Phosphorothioate-modified CG-rich oligodeoxynucleotide 1668 (5'-TCC ATG ACG TTC CTG AAT AAT-3', SEQ ID NO:8) was synthesized by Microsynth (Balgach, Switzerland).

**Immunization schedules**. The different immunization schedules (s1 to s6) were designed to deliver a fixed cumulative dose of 125 µg gp33 (KAVYNFATM, SEQ ID NO:3) peptide or influenza matrix peptide (GILGFVFTL, SEQ ID NO:6; Falk, K. et al., Immunology 82, 337-342, 1994 , which is incorporated herein by reference in its entirety) and 12.5 nmol CpG 1668 over a time frame of one to four days (Table 1). Note that schedules 3 (s3) and 4 (s4) follow an exponentially decreasing or increasing pattern at 5-fold dilution steps, respectively. Immunization with influenza matrix peptide was done with the same cumulative dose of 125 µg and followed the same schedule.

**Adoptive transfer experiments.** 1 x 10⁶ TCR-transgenic T cells were resuspended in 250 µl of PBS and injected into the tail vein of sex-matched C57BL/6 mice in order to increase precursor T cell frequencies and facilitate assessment of the immune response. One day later, the recipients were subcutaneously vaccinated in the neck region with varying doses of gp33 peptide mixed with cytosine-guanine oligodeoxynucleotide (CpG ODN) as indicated in Table 1. Alternatively, mice were intravenously infected with LCMV-WE strain (250 pfu). Immunization with influenza matrix peptide was done with the same cumulative dose of 125 µg following the same schedules.

**FACS analysis**. For FACS analysis of surface antigens, RBC-free single-cell suspension of blood, spleens or lymph nodes were prepared. The cells were incubated on ice for five minutes with anti-CD16/CD32 for Fc-receptor blocking, and stained with PE-labeled gp33 MHC class-I tetramer (gp33/H-2Db) for 15 minutes at 37°C followed by staining for other surface antigens on ice for 20 minutes. All stainings were made in PBS/FCS 2% with 0.01% sodium azide. For intracellular staining of IFN-γ, single-cell suspensions were cultured *in vitro* with 2 x 10⁻⁶ M gp33 peptide and 10 µg/ml Brefeldin A (Sigma, Buchs, Switzerland) in complete medium for four hours. Lymphocytes were then surface stained as above, fixed in protein-free PBS/PFA 1% for 10 minutes, permeabilized in PBS/NP40 0.1% for three minutes on ice, and finally incubated with anti-IFN-γ. antibodies in PBS/FCS 2% on ice for 35 minutes. Samples were acquired on a FACSCalibur and analyzed using CellQuest software from BD were acquired on a FACSCalibur and analyzed using CellQuest software from BD Biosciences (San Jose, CA) or FlowJo software from TreeStar Inc. (Ashland, OR). All other antibodies were purchased from BD Pharmingen (San Diego, CA).

**Assessment of anti-viral immunity in** ***vivo**.* Vaccinated female C57BL/6 mice were infected intraperitoneally with 1.5 x 10⁶ pfu vacc-gp. Five days later, ovaries were isolated and the vaccinia titers were determined on BSC 40 cells as described by Kundig, T.M. et al., in J Virol 67, 3680-3; 1993, *supra.* Alternatively, the mice were infected with 250 pfu LCMV-WE, and viral titers in spleens were determined on MC57 cells (Battegay *et al.,* 1991, *supra*)*.*

**Cytotoxicity assay and cytokine secretion analysis.** 1 x 10⁵ transgenic gp33-specific T cells were cultured for six days in 24-well plates together with syngeneic irradiated feeder cells (2 x 10⁶ cells/well; 2000 rads) and pulsed with the indicated amounts of gp33 peptide. Effector cells were then resuspended in 300 µl fresh medium and threefold dilutions were made. EL-4 cells were pulsed with 10⁻⁶ M gp33 peptide and used as target cells in a five hour ⁵¹Cr release assay (Bachmann, M.F. et al., Eur J lmmunol 24, 2228-36, 1994, *supra*)*.* Radioactivity in cell culture supernatants was measured with a Cobra II Counter (Canberra Packard, Downers Growe, IL). Nonradioactive culture supernatants were assessed daily for IFN-γ, IL-2 and IL-10 concentrations. The cytokine analysis was performed using beads-multiplex-assays and flow cytometry.

**Preparation of bone-marrow derived dendritic cells for vaccination**. Bone-marrow cells were isolated from femurs of young C57BL/6 mice and seeded at 2×10⁶ cells in 100-mm dish in 10 ml supplemented medium with 50 ng/ml rmGM-CSF and 25 ng/ml rmIL-4 (R&D Systems, Minneapolis, MN). On day seven, cells were harvested, and the DCs were purified by positive selection using anti-CD11c microbeads (Miltenyi Biotec, Bergisch Gladbach, Germany). Purified cells were plated in six-well plates and stimulated overnight with 2 µM CpG ODN 1668. The DC phenotype was assessed by flow cytometry using a panel of labeled mAb against CD80, CD86, CD40, CD11c, and a mouse lineage antibody cocktail (CD3e, Cd11b, CD45R/B220, Ly-76, Ly-6G, Ly-6C). All antibodies were obtained from BD Pharmingen. Subsequently, the DCs were pulsed with the HPV E7 (aa49-57) peptide (RAHYNIVTF, SEQ ID NO:7) at 10 µg/ml at 37° C for two hours. The DCs were washed three times with PBS before administration of 25 µL of the DC bilaterally into the inguinal lymph nodes of anesthetized C57/B6 mice (Johansen et al. 2005a. Eur J Immunol 35:568-574 , which is incorporated herein by reference in its entirety). Groups of ten mice received either a single bolus injection of DCs (1.11×10⁵) on Day 1 (s1), or injections of exponentially or increasing number of DCs (10³, 10⁴, and 10⁵) on Days 1, 3 and 6 (s4).

**E7 Tetramer Analysis**. PBMCs were isolated from mice (n=10) on day 17 and mononuclear cells were separated from RBCs following density centrifugation (Lympholyte Mammal, Cedarlane Labs). The E7₄₉₋₅₇ specific CTL response was quantified by staining cells with H-2Db HPV16 E7 (RAHYNIVTF, SEQ ID NO:7)-PE MHC tetramer (Beckman Coulter) and FITC-conjugated anti-CD8a (Ly-2) (BD Pharmingen) mAb at 40°C for one hour. Data were collected using a FACSCalibur and analyzed using CellQuest software.

**ELISPOT Analysis**. For quantification of IFN-γ producing cells, spleens were isolated on day 21 and single cell suspensions were prepared (n=7). Mononuclear cells were isolated by density centrifugation and re-suspended in serum free HL-1 complete medium containing non-essential amino acids, sodium pyruvate, glutamine pen-strep, beta-mercaptoethanol, and HEPES. Triplicates of 2.5×10⁵ splenocytes were incubated with 10 µg/well of HPV16 E7 peptide at 37°C for 72 hours in 96-well filter-membrane plates (Multi-screen IP membrane 96-well plate, Millipore). ELISPOTs were quantified using ELISpot Reader and software from AID (Strassberg, Germany) after 24 hours development using coating and detection IFN-γ antibodies from U-Cytech biosciences (Utrecht, The Netherlands).

**Tumor Protection Studies**. On day 21 (15 days following the last injection of DCs), three mice from each vaccinated group and seven naïve C57/B6 mice were challenged with 10⁵ cells of the HPV transformed tumor cell line C3.43, (Feltkamp et al. 1993. Eur J Immunol 23:2242-2249; Feltkamp et al. 1995. Eur J Immunol 25:2638-2642, each of which is incorporated herein by reference in its entirety), and cultured in DMEM, 10% FBS, 2 mM L-glutamine, and 50 µM 2-mercaptoethanol. The cells were administered subcutaneously in the left flank. Tumor progression was monitored by caliber measurements (mm) and tumor volumes calculated.

**Statistical Analysis**. The student's t-test assuming equal variances was performed and data was considered significant with a non-paired two tailed p value lower than 0.05. Non-parametric or non-normally distributed data were analyzed using the Mann Whitney U test or by Kurskal-Wallis ANOVA. The comparison of Kaplan-Meier survival curves were performed using the log rank test.

### EXAMPLE 2: EXPONENTIALLY INCREASING ANTIGENIC STIMULATION ENHANCES CD8⁺ T CELL RESPONSE

It was investigated whether a T cell response can be enhanced by increasing antigenic stimulation. In a first experiment, 1 x 10⁶ transgenic gp33-specific T cells were transferred into C57BL/6 wild type recipient mice to increase precursor T cell frequencies and facilitate assessment of the immune response.

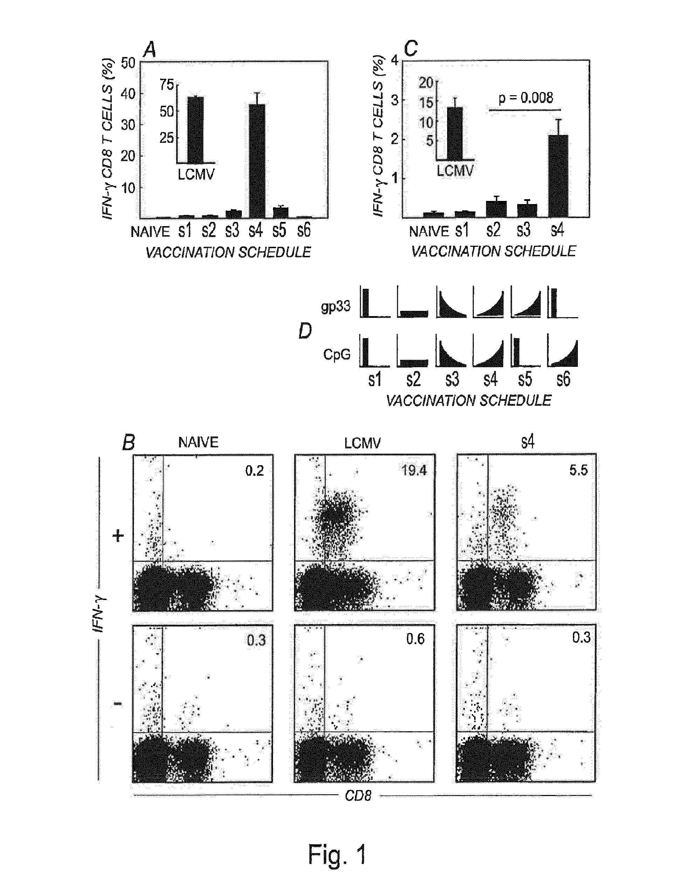

All mice were immunized with the same cumulative dose of gp33 peptide mixed with CpG ODN (in total 125 µg gp33 and 12.5 nmol CpG), using different vaccination protocols as disclosed in **FIG. 1D** and Table 1: s1) one single dose in a bolus injection at day 0; s2) four equal doses over four days; s3) decreasing doses over four days; and s4) increasing doses over four days. Additionally, groups of mice were immunized with a single dose of CpG followed by exponentially increasing doses of gp33 peptide (s5), or with a single dose of gp33 followed by exponentially increasing doses of CpG (s6). Mice intravenously infected with 250 pfu of LCMV virus on day zero served as a positive control. On day 6 (FIG. 1A, day 12 (FIG. 1B) and day 8 (FIG. 1C), CD8⁺ T cell responses were quantified by intracellular IFN-γ staining of blood lymphocytes restimulated with gp33 peptide in vitro. FIG. 1B depicts representative FACS examples of the analysis on day 12.

CpG ODN was chosen as the adjuvant since they strongly enhance CD8⁺ T cell responses (Krieg, A.M., Annu Rev Immunol 20, 709-60, 2002; Schwarz, K. et al., Eur J Immunol 33, 1465-70, 2003, each of which is incorporated herein by reference in its entirety). Phosphorothioate stabilized ODN are cleared from plasma with a half-life of 30-60 minutes (Farman, C.A. & Kornbrust, D.J., Toxicol Pathol 31 Suppl, 119-22, 2003, which is incorporated herein by reference in its entirety). However, in tissues CpG ODN are relatively stable with a half-life of 48 hours (Mutwiri, G.K., et al., J Control Release 97, 1-17, 2004, which is incorporated herein by reference in its entirety). Further, it is noted in the literature that within 60 minutes serum proteases degrade the free peptides below detection levels (Falo, L.D., Jr., et al., Proc Natl Acad Sci USA 89, 8347-50, 1992; Widmann, C., et al., J Immunol 147, 3745-51, 1991, *supra*).

The data shows that immunization leading to CD8⁺ T cell responses of a magnitude comparable to infection with LCMV wild type required that both gp33 and CpG were administered in an exponentially increasing fashion. Immunization using uniform daily doses of gp33 and CpG induced the second strongest CD8⁺ T cell responses which was, however, significantly weaker than dose-escalating stimulation (p=0.0001 on day six). If either one of the vaccine components was delivered as a single dose, the efficacy of immunization was significantly reduced but significant compared to the naive control (2.23 ± 0.84 % vs. 0.19 ± 0.12 % p=0.02 on day six).

Similar observations were made in naïve wild type mice that did not receive TCR transgenic cells (FIG. 1C). C57BL/6 mice immunized with exponentially increasing vaccine (gp33 and CpG) doses showed significantly enhanced induction of CD8⁺ T cells (2.1 ± 0.4 %) compared to the other vaccination protocols (p<0.008), which induced barely detectable frequencies of specific CD8⁺ T cells. None of the tested groups showed measurable immune responses on day four (data not shown). These results demonstrate that, independent of the overall dose, the kinetics of the vaccination is a key parameter of immunogenicity.

None of the tested groups showed measurable immune responses on day four (data not shown). Overall, these results showed that independent of the overall dose, the kinetics of the antigen and the adjuvant are key parameters of immunogenicity.

In the figures: s1: single dose of gp33 peptide and CpG; s2: equivalent doses of gp33 peptide and CpG; s3: exponentially decreasing doses of gp33 peptide and CpG; s4: exponentially increasing doses of gp33 peptide and CpG; s5: exponentially increasing doses of gp33 peptide and an initial single dose of CpG; s6: initial single dose of gp33 peptide and exponentially increasing doses of CpG; naive: untreated mice; LCMV: mice intravenously immunized with 250 pfu of LCMV on day zero. Values represent the means and SEM of four mice per group. One representative experiment of three similar experiments is shown.

FIG. 1B is a representative FACS example of the analysis on day 12. Upper panel: Re-stimulation with gp33 peptide, lower panel: Control staining without gp33 re-stimulation (lower panel).

| **Table I: Immunization Schedules** | | | | | | |
|---|---|---|---|---|---|---|
| **Schedule** | | **Day 0** | **Day 1** | **Day 2** | **Day 3** | **Cumulative dose** |
| **s1** | **Peptide (µg)** | **125** | **0** | **0** | **0** | **125 µg** |
| | **CpG (nmol)** | **12.5** | **0** | **0** | **0** | **12.5 nmol** |
| **s2** | **Peptide (µg)** | **31.25** | **31.25** | **31.25** | **31.25** | **125 µg** |
| | **CpG (nmol)** | **3.1** | **3.1** | **3.1** | **3.1** | **12.4 nmol** |
| **s3** | **Peptide (µg)** | **100** | **20** | **4** | **0.8** | **125 µg** |
| | **CpG (nmol)** | **10** | **2** | **0.4** | **0.08** | **12.5 nmol** |
| **s4** | **Peptide (µg)** | **0.8** | **4** | **20** | **100** | **125 µg** |
| | **CpG (nmol)** | **0.08** | **0.4** | **2** | **10** | **12.5 nmol** |
| **s5** | **Peptide (µg)** | **0.8** | **4** | **20** | **100** | **125 µg** |
| | **CpG (nmol)** | **12.5** | **0** | **0** | **0** | **12.5 nmol** |
| **s6** | **Peptide (µg)** | **125** | **0** | **0** | **0** | **125 µg** |
| | **CpG (nmol)** | **0.08** | **0.4** | **2** | **10** | **12.5 nmol** |

### EXAMPLE 3: ENHANCEMENT OF CD8⁺ T CELL RESPONSE IS INDEPENDENT OF T CELL HELP

The role of T-help with respect to CD8⁺ T cell priming is well known in the art. Th-epitopes can be crucial for functional CD8⁺ T cell immunity (Johansen et al., Eur J Immunol., 34, 91-97, 2004; Shedlock and Shen, Science, 300, 337-339, 2003; Sun and Bevan, Science, 300, 339-342, 2003, each of which is incorporated herein by reference in its entirety). On the other hand, in situations where the precursor frequency is high, CD8⁺ T cell responses are less Th dependent (Mintern et al., J Immunol., 168, 977-980, 2002 , which is incorporated herein by reference in its entirety) especially when using a strong immunogen, e.g., LCMV gp33. Moreover, the route of administration can also effect the requirement for T-help (Bour et al., J Immunol., 160, 5522-5529, 1998 , which is incorporated herein by reference in its entirety). Therefore, the Th-dependency of CTL very much depends on the experimental setting. Based on this hypothesis, the inventors examined whether enhancement of the CD8⁺ T cell response was independent of T cell help by vaccination with exponentially increasing vaccine doses.

Mice were immunized with exponentially increasing vaccine doses as in the above-described protocols (see Table 1) using a mixture of the class-1 LCMV gp33 (aa33-41) peptide and the class-II LCMV gp61 (aa61-80) Th-epitope of LCMV. Enhancement of the CD8⁺ T cell response by vaccination with exponentially increasing vaccine doses was observed to be independent of T cell help, since the same effects of dose kinetics on CD8⁺ T cell responses were obtained in mice immunized with the above-described protocols (data not shown).

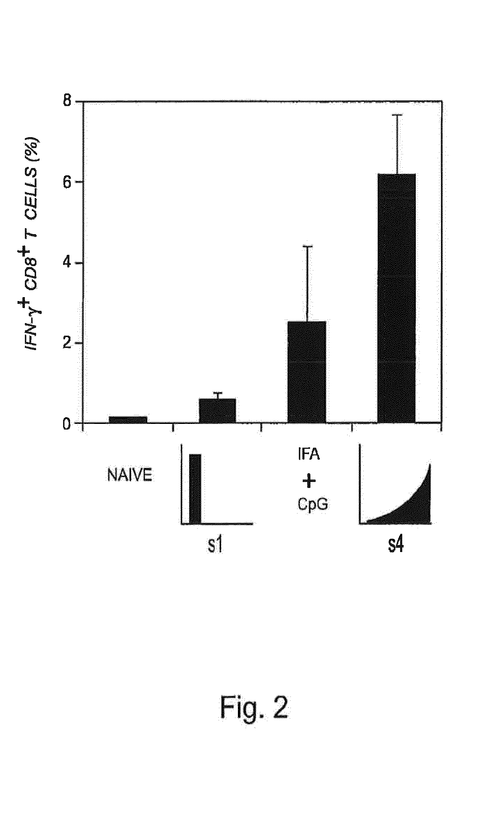

To examine whether the exponential immunization observed above can be achieved with other peptides, the analysis was expanded to another peptide derived from the influenza matrix protein which binds to the human HLA A2.1 class-I molecule (Falk, K. et al., Immunology: 82, 337-42, 1994, *supra).* Transgenic mice expressing HLA A2.1 (HHD; Pascolo, S. et al., J. Exp. Med. 185, 2043-51, 1997, *supra)* were subcutaneously immunized with an influenza-matrix peptide (GILGFVFTL, SEQ ID NO:1) and CpG ODN. The immunization schedule was as described in Table 1 (above). The vaccine was given as one single bolus (125 µg peptide and 12.5 nmol CpG, s1) or the same total dose was administered over four days in a dose-escalating manner (s4). Incomplete Freund's adjuvant (IFA), a mineral oil that releases the antigen slowly, was used as a positive control (Miconnet, I. et al., J Immunol., 168, 1212-8, 2002; Speiser, D.E. et al., J Clin. Invest. 115, 739-46, 2005; Aichele, P., et al., J Exp. Med. 182, 261-6; 1995, each of which is incorporated herein by reference in its entirety). After eight days CD8⁺ T cells were analyzed for IFN-γ production after *in vitro* restimulation of blood lymphocytes with peptide (means ± SEM; n=3-4). It was observed that exponentially increasing vaccine doses generated higher frequencies of IFN-γ-producing cells (6.2% ± 1.5) than did the treatment with a single dose of peptide and CpG (0.6% ± 0.2) or peptide and CpG emulsified in IFA (2.5% ± 1.9); (**FIG. 2**).

### EXAMPLE4: ANTIGEN ADMINISTRATION FOR FOUR OR MORE DAYS INDUCES MAXIMAL CD8⁺ T CELL RESPONSES

As shown in the above Examples, antigen that exponentially increased over a time period of four days induced significantly stronger T cell responses than a bolus injection or daily injections of uniform vaccine doses. Experiments were therefore conducted to examine whether further prolongation of antigen presentation would enhance the responses even further. Groups of C57BL/6 mice were subcutaneously immunized with the same total dose of gp33 peptide and CpG (125 µg p33 and 12.5 nmol CpG), but following different exponential kinetics, by injecting the dose as a bolus or over four, six or eight days (**FIG**. **3A**) with peaks at day zero (bolus), three, five or day seven.

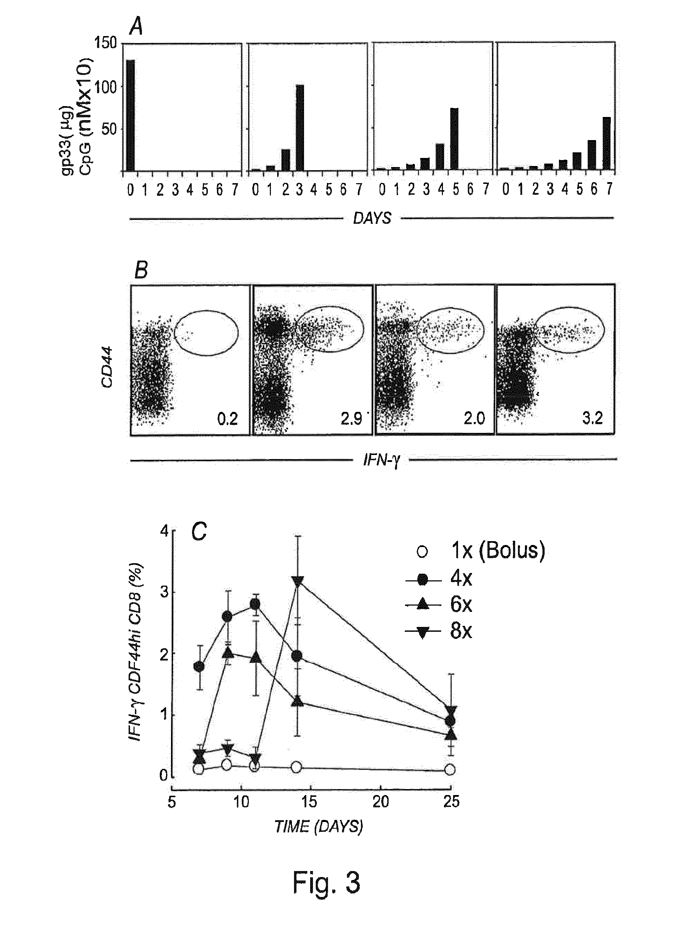

At different time points after the last injection, blood lymphocytes were isolated and re-stimulated *in vitro* with gp33 peptide for determination of CD44 expression and intracellular IFN-γ by flow cytometry. As illustrated in FIG. 3B, in comparison to a single bolus injection, injections over four, six or eight days resulted in comparable significantly enhanced frequencies of specific CD8⁺ T cells at the height of the immune response, which was four to seven days after the last injection. The FACS density blots depict the frequencies of CD44hi and IFN-γ-producing CD8-positive lymphocytes as measured by FACS at the peak of the immune response, and the numbers show the mean percentage of IFN-γ-producing CD44hi CD8⁺ T cells. One out of two similar experiments is shown (n=3-4).

The mean percentage of IFN-γ-producing CD44hi CD8⁺ T cells is also depicted as a function of time (FIG. 3C). One out of two similar experiments is shown (n=3-4). Antigen kinetics that peaked on day four induced significantly stronger CTL responses compared to a shorter or a longer antigen profile which induced significantly weaker responses. Moreover, there was no statistical difference in the number of resting memory cells as measured four weeks after the last injection. A biological reason for these observations can be that proliferation and differentiation of CD8⁺ T cells into effector cells takes several days, and it would be difficult for the immune system to even compete with a pathogen that overwhelmingly infects the host within one or two days. On the other hand, pathogens that replicate for prolonged periods of time cause more damage when they are constantly fought by CTL.

### EXAMPLE 5: EXPONENTIALLY INCREASING ANTIGENIC STIMULATION ENHANCES PROTECTIVE ANTIVIRAL RESPONSE

To further elucidate the functional relevance of the above observations, female wild type C57BL/6 mice were immunized with fixed cumulative doses of gp33 peptide and CpG according to different regimens (s1-s4 as shown in Table 1) and then challenged with LCMV or a recombinant vaccinia virus expressing the LCMV glycoprotein (vacc-gp) at time points when T cell responses are already in a contraction or memory phase (Kaech, S.M., et al., Nat Rev Immunol 2, 251-62, 2002 , which is incorporated herein by reference in its entirety). Protection against both viruses is exclusively dependent on CD8⁺ T cells (Binder, D. and Kundig, T.M., J Immunol 146, 4301-7, 1991; Kundig, T.M. et al., Proc. Natl. Acad. Sci., USA: 93, 9716-23, 1996, each of which is incorporated herein by reference in its entirety).

Mice (n=4) were immunized with exponentially increasing amounts (s4) or with a bolus injection (s1) of gp33 peptide and CpG as described above (Table 1). Negative control mice were left untreated (naïve) and positive control mice were infected with LCMV (250 pfu). The mice were bled on day 10 and day 30 for analysis of gp33-specific effector or memory CTLs using gp33-MHC-tetramers and flow cytometry (**FIG**. **4A**) or on day 30 for analysis of IFN-γ-producing CD8⁺ T cells after re-stimulation *in vitro* with gp33 (**FIG**. **4B**). **FIG**. **4A** depicts gp33-tetramer-positive CD44hi expression on day 10 and day 30, and from left to right, Naïve, s1, s4 and LCMV. In line with the above-described results, exponentially increasing doses of peptide (gp33) and CpG induced significantly higher frequencies of IFN-γ-producing effector and memory cells (**FIG**. **4B**) and gp33-tetramer-positive memory (CD44^{hi}) cells (**FIG**.**4A**) than did a single-shot vaccination. On day 30, all mice were challenged by intraperitoneal injection with 250 pfu LCMV. Four days later, viral titers were measured in spleens. On day 30, the mice were challenged intraperitoneally with 250 pfu LCMV. Four or five days later, spleens or ovaries were harvested for determination of LCMV. While bolus (s1)-vaccinated mice were not significantly protected against viral replication (**FIG**. **4C**), exponentially increasing vaccination induced significant protection inhibiting LCMV titers approximately 10- to 20-fold when compared to naive or bolus-vaccinated mice (p<0.01).

In another set of experiments, C57BL/6 mice were immunized using different regimes and then challenged intravenously on day 8 (**FIG**. **4D**) or 24 (**FIG**. **4E**) with 1.5×10⁶ pfu of the recombinant vaccinia virus (vacc-gp). Five days thereafter, vacc-gp replication was determined in ovaries (**FIG**. **4D and 4E**). Again, only mice immunized in a dose-escalating fashion were able to mount significantly protective CD8⁺ T cell responses, inhibiting viral replication on average two to three orders of magnitude better than the other peptide immunization protocols.

These results therefore, attested to the biological relevance of the kinetics of antigen presentation during immunization.

### EXAMPLE 6: THE NUMBER OF ACTlVATED APCs IS NOT DEPENDENT ON THE TYPE OF STIMULATION KINETICS

To test whether the immunization kinetics affected the activation status and the numbers of activated APCs, C57BL/6 mice were immunized with gp33 peptide and CpG according to the immunization protocol s1 (bolus injection) and s4 (exponentially increasing doses) as described in Figures 1-3 and in Table 1. The vaccines were administered subcutaneously in the inguinal region. After one, four, six and eight days, the inguinal lymph nodes were removed and single cell suspensions thereof where analyzed by flow cytometry for the expression of the DC marker CD11c, as well as CD86 and the MHC class II marker I-Ab (**FIG**. **5A**). The results are shown expression as mean fluorescence relative to naïve controls (day zero). The results suggested that the different kinetics did neither crucially affect the numbers of DCs in the draining lymph nodes, nor their activation status as monitored by MHC-class II (I-Ab) and CD86 expression (**FIG**. **5A**). However, although the peaks of DC numbers and activation were comparable, they were separated in time by 2-3 days. DC activation reached its maximum one day after the maximal CpG dose, independent of the type of immunization kinetics.

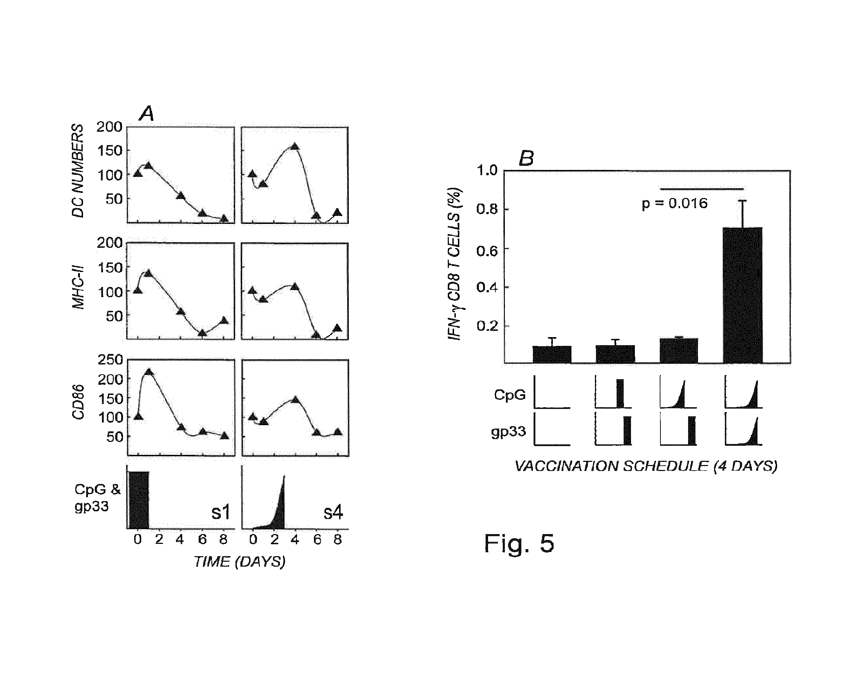

Therefore, the possibility that exponentially increasing vaccination was optimal for CD8⁺ T cell induction, simply because the antigenic peptide is delivered at a time point when DCs are the most activated, was tested. If this were true, administration of a high dose of peptide as a bolus one day after a bolus injection of CpG or one day after the last dose of CpG given in an exponentially increasing pattern over four days, should result in a CD8⁺ T cell response comparable to giving both peptide and CpG in a dose-escalating fashion. In a separate experiment, mice were immunized with gp33 peptide and CpG according to modified protocols as depicted (**FIG**. **5B**). One group received a CpG bolus on day three and a gp33 peptide bolus on day four. One group received exponentially increasing CpG doses on days zero to three followed by a gp33 peptide bolus on day four. The last group received exponentially increasing doses of gp33 peptide and CpG on days one to four as described above (s4). The frequency of IFN-γ-producing CD8⁺ T cells was measured in peripheral blood on day 10. The results show means and SEM of one out of two comparable experiments (n=3).). As evident from **FIG. 5B****,** pre-stimulation of APCs with CpG resulted in gp33-specific immune responses that were significantly lower than the response produced by the exponentially increasing pattern of administration of gp33 and CpG together (p=0.016)

### EXAMPLE 7: EXPONENTIALLY INCREASING ANTIGENIC STIMULATION FAVORS PROLONGED T CELL STIMULATION

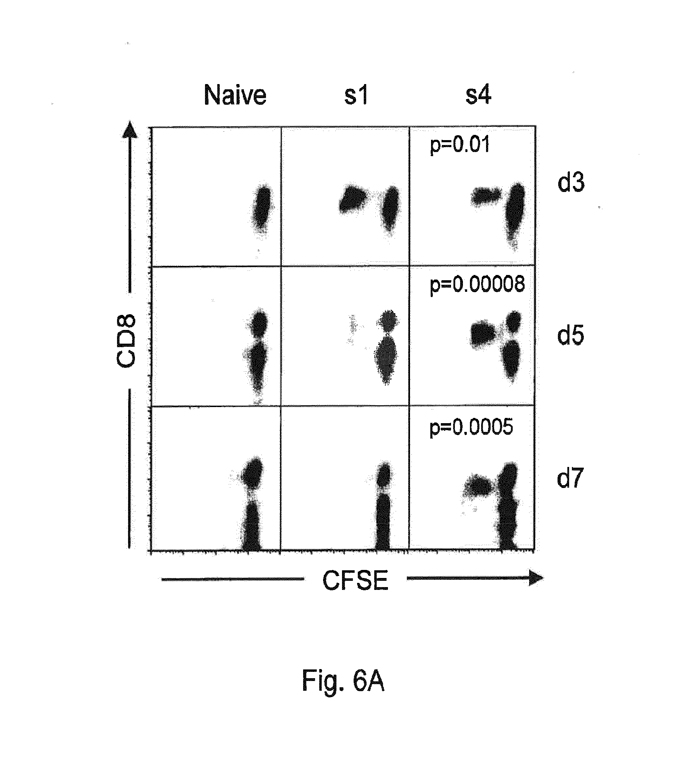

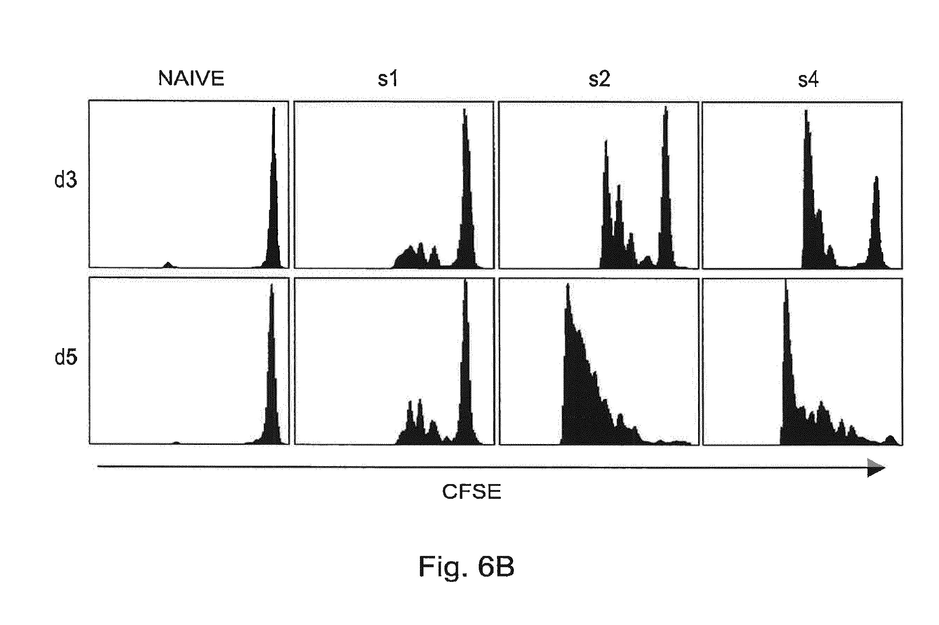

To test how the kinetics of immunization affected the proliferation of CD8⁺ T cells, mice were injected with a single dose (s1), uniform daily doses (s2) or with exponentially increasing doses (s4) of gp33 peptide and CpG as described above and in Table 1. One group of mice was left untreated as a negative control. To monitor proliferation, all mice received 10⁷ or 1.5×10⁶ CFSE-labeled splenocytes from transgenic TCR318 mice intravenously one day before the first immunization. At different time points, lymphocytes were isolated by tail bleeding and analyzed for CD8 expression and CFSE staining by flow cytometry. The p values indicate statistical differences between the s1 and s4 schedules with regard to the percentage of CFSE-labeled CD8⁺ T cells that have entered division. The results show one of two comparable experiments. A bolus injection of gp33 peptide and CpG triggered CFSE-labeled CD8⁺ T cells to divide three days after the immunization (**FIGs. 6A** **and** **6B**). Proliferation could be detected already after two days (**FIG. 6B**). On day five, precursor cells still entered division although to lower extent than at day three, and by day seven, the CFSE-labeled cells had ceased to enter new divisions. In contrast, the exponentially increasing stimulation markedly prolonged the T cell proliferation. Cells entering division could be determined as early as three days post priming, despite not yet having received the whole immunization regime, and the division prolonged through days five and seven (**FIG. 6B**). Even on day nine, proliferation could still be observed (not shown). Moreover, the division index, i.e., the average number of the division undergone, was significantly higher (p<0.05 by Mann Whitney) for s4 than for s2.

### EXAMPLE 8: EXPONENTIALLY INCREASING NUMBERS OF PEPTIDE PULSED DCs ENHANCE CD8⁺ T CELL RESPONSES

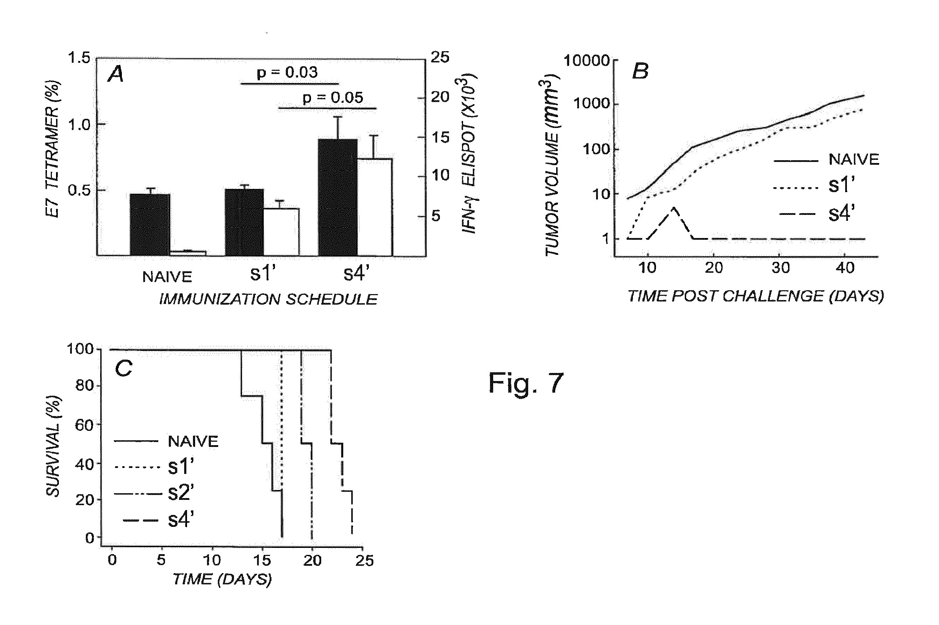

To investigate the contribution of different numbers of APCs, C57BL/6 mice were immunized with the same total numbers of peptide-pulsed DCs, but using different kinetics. Bone-marrow derived DCs were loaded with the HPV E7 (aa49-57, RAHYNIVTF, SEQ ID NO:2) peptide, and a total of 1.11×10⁵ cells were injected into the inguinal nodes as a bolus on day one (s1), or the same total number of cells was administered in an increasing (s4) pattern on days one (10³ cells), three (10⁴ cells), and six (10⁵ cells). Furthermore, the vaccines were administered intralymphatically in order to ensure a constant total number of DCs available for T cell priming. Naïve mice were used as negative controls. On day 17 and day 22, the frequency of E7-tetramer positive CD8⁺ T cells in peripheral blood (**FIG**. **7A** (■)) was analyzed by flow cytometry. The values represent means and SEM (n=10). IFN-γ ELISPOTs (**FIG. 7A** (□) were analyzed from spleens (n=7). The values represent means and SEM (n=7). On day 21, three vaccinated mice and ten naïve mice were challenged with the HPV-transformed tumor cell line G3.43 (**FIG. 7B**). Tumor progression was monitored by caliber measurements (mm) from which tumor volumes were calculated. Survival after challenge was studied in C57BL/6 mice (n=4) immunized by s.c injection of DCs loaded with the VSV np52 peptide (**FIG. 7C**). Log-rank tests of Kaplan Meier curves: s4 ≠ s2: p= 0.0084; s2 ≠ s1 : p= 0.0082; s1 ≠ Naive: p= 0.401.

Exponentially increasing doses (s4) again induced a higher number of antigen-specific CD8⁺ T cells than a bolus injection of the vaccine (s1). This was evident for both the frequencies of MHC-E7-tetramer positive (**FIG. 7A** (■)) and of IFN-γ-producing (**FIG. 7A** (□)) CD8⁺ T cells measured on days 17 and 22, respectively. In correlation to the strength of the measured CD8⁺ T cell response, mice vaccinated with the dose-escalating protocol rejected a challenge with the HPV-transformed tumor cell line C3.43 (**FIG. 7B**). In contrast, mice vaccinated merely with a single bolus were not protected.

By the same token, mice immunized with the (s4) protocol of DCs loaded with the VSV np52 peptide showed improved survival after a challenge with mouse lymphoma cells EL-4 transfected to express the VSV nucleoprotein (**FIG. 7C**). C57BL/6 mice were immunized by s.c injection of DCs loaded with the VSV np52 peptide (n=4). 1.11x10⁵ DCs were given as a bolus on day 1 (s1) or as equal (s2) or dose escalating (s4) doses on days 1, 3, and 6. Naïve mice were used as controls. On day 14, all mice were challenged with dose 10⁶ EL-4 N.1 cells i.p (Kundig et al., J Immunol. 150, 4450-4456, 1993, *supra*). The data illustrates that the survival of (s4) immunized mice was also significantly better than mice immunized according to the (s2) protocol with DCs given in the uniform numbers on the three days (p=0.084).

Thus, exponentially increasing vaccination proved more immunogenic than bolus vaccination. As these experiments keep DC activation at the same level throughout immunization, and the total number of DCs is the same, this confirms that the kinetics of appearance of activated peptide presenting DCs determines the strength of the CD8⁺ T cell response. The inventors therefore concluded that the synchronization of DC numbers to the frequency of specific T cells enhances the final burst size of the T cell response. While the low frequency of specific T cells during the early response can be efficiently stimulated with a low number of antigen pulsed DCs, it appears important to re-stimulate the high frequency of specific T cells during the later primary response with a high number of DCs.

### EXAMPLE 9: EXPONENTIALLY INCREASING ANTIGENIC STIMULATION ENHANCED IL-2 PRODUCTION IN T CELLS: THE IMPACT OF ANTIGEN KINETICS AT THE LEVEL OF T CELL CLONES

The inventors next investigated whether the observations in the above Examples could be explained at the level of a T cell clone, or whether they were the result *of in vivo* T cell selection processes involving T cell clonotypes of differential affinity, avidity and functionality.

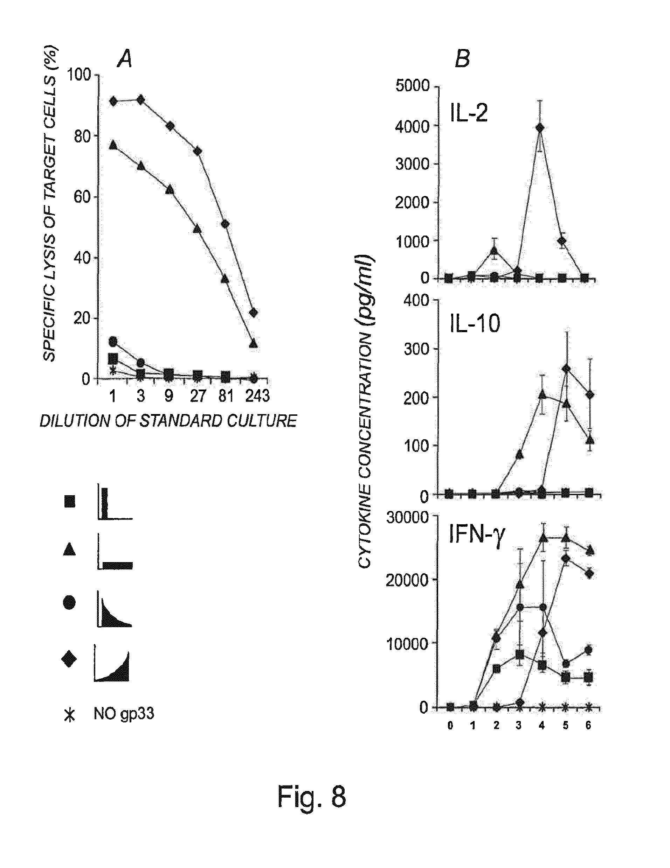

1x10⁵ TCR-transgenic T cells were co-cultured with 2x10⁶ irradiated syngeneic splenocytes serving as feeder cells. T cells expressing a transgenic T cell receptor recognizing gp33 in context of D^{b} were stimulated *in vitro* with the same total dose of antigen, but corresponding to various profiles, *i.e*., either with a bolus of 10⁻⁹ M gp33 on day zero (■); with exponentially increasing gp33 doses of 10⁻¹², 10⁻¹¹, 10⁻¹⁰, and 10⁻⁹ M at days zero, one, two and three, respectively over 4 days (◆); with the same gp33 dose of 0.25x10⁻⁹ M every day for four days (▲); or with exponentially decreasing gp33 doses of 10⁻⁹,10⁻¹⁰, 10⁻¹¹ and 10⁻¹² M at days zero, one, two and three, respectively (II). Control cells without gp33 stimulation are illustrated as (*). IL-2, IL-10 and IFN-γ were determined daily in supernatants (**FIG. 8B**), and after six days CTL activity was determined in a five-hour ⁵¹Cr release assay (**FIG. 8A**). The values represent means of duplicate (**FIG. 8A**) and triplicate (**FIG. 8B**) cultures.

Similar to the *in vivo* findings, exponentially increasing immunogen doses induced the strongest CTL response, followed by daily administrations of the same gp33 dose, while administration of the immunogen as a bolus or with a decreasing dose profile generated weaker CTL responses. The difference was even greater when cells were stimulated with one tenth of the peptide doses. The CTL activity correlated with the production of IL-2 (**FIG. 8B****, top panel**). Exponentially increasing immunogen doses induced the highest amounts of IL-2, while constant daily immunogen doses induced much less IL-2. Constant antigenic stimulation induced high amounts of IL-10 with an earlier onset as compared to exponentially increasing antigenic stimulation (**FIG. 8B****, middle panel**). IFN-γ was transiently produced at an early stage in cells stimulated with an immunogen bolus or exponentially decreasing amounts of immunogen (**FIG. 8B****, bottom panel**). In contrast, daily stimulation by constant or exponentially increasing doses induced secretion of higher amounts of IFN-gamma by specific T cells.

Therefore, as observed, continued and sufficient antigenic stimulation was necessary to maintain IFN-γ production. The fact that exponentially increasing antigenic stimulation appears to produce less IFN-γ than constant daily doses can be explained by high *in vitro* stability of IFN-γ and consecutive accumulation due to earlier IFN-γ production.

Taken together, *in vitro* stimulation of clonotypic T cells with exponentially increasing immunogen doses produced higher amounts of IL-2 and IFN-γ and stimulated IL-10 at a later time point than all other antigen profiles. These observations are consistent with the conclusion that the enhanced immune response brought about by increasing antigenic stimulation operates at a clonotypic level. These phenomena have also been shown to be accompanied by higher T cell avidity, which is crucial for efficient interaction between T cells and dendritic cells (Bousso and Robey. 2003. Nat Immunol 4: 579-585, which is incorporated herein by reference in its entirety).

Production of IL-2 is a hallmark of CD4⁺ and CD8⁺ T cell activation and plays a key role in regulating several stages of the T cell response. Engagement of the TCR (signal 1) and co-stimulatory molecules (signal 2) induces only limited clonal expansion of T cells. Extensive amplification of T cells as well as differentiation into effector cells to mount a productive T cell response requires signalling via the IL-2R (signal 3; Malek, T.R. and Bayer, A.L., Nat. Rev. Immunol., 4, 665-74, 2004, which is incorporated herein by reference in its entirety) and autocrine IL-2 production by CD8⁺ T cells is a key driver of in vivo CD8⁺ T cell expansion (Malek, T.R. & Bayer, A.L., Nat. Rev. Immunol., 4, 665-74, 2004, *supra*; D'Souza, W.N., et a/., J Immunol., 168, 5566-72, 2002, which is incorporated herein by reference in its entirety). On the other hand, IL-10 is a main inhibitor of T cell proliferation mostly via modulation of dendritic cells (Moore, K.W., et al., Annu. Rev. Immunol., 19, 683-765, 2001, which is incorporated herein by reference in its entirety). Thus, the in vivo data of the present invention indicated that, at a clonal level, T cells are capable of decoding the kinetics of antigen exposure.

### EXAMPLE 10: LINEARLY INCREASING ANTIGENIC STIMULATION ENHANCES CD8⁺ T CELL RESPONSES

An investigation is conducted to determine whether a T cell response can be enhanced by increasing antigenic stimulation. In an experiment, 1 x 10⁶ transgenic gp33-specific T cells are transferred into C57BL/6 wild type recipient mice to increase precursor T cell frequencies and facilitate assessment of the immune response.

All mice are immunized with the same cumulative dose of gp33 peptide mixed with CpG ODN (in total 125 µg gp33 and 12.5 nmol CpG), using different vaccination protocols as follows: s1) one single dose in a bolus injection at day 0; s2) four equal doses over four days; s3) linearly decreasing doses over four days; and s4) linearly increasing doses over four days. Additionally, groups of mice were immunized with a single dose of CpG followed by linearly increasing doses of gp33 peptide (s5), or with a single dose of gp33 followed by linearly increasing doses of CpG (s6). Mice intravenously infected with 250 pfu of LCMV virus on day zero serve as a positive control. On day 6, day 12 and day 8, CD8⁺ T cell responses are quantified by intracellular IFN-γ staining of blood lymphocytes restimulated with gp33 peptide *in vitro.*

CpG ODN is chosen as the adjuvant since they strongly enhance CD8⁺ T cell responses (Krieg, A.M., Annu Rev Immunol 20, 709-60, 2002; Schwarz, K. et al., Eur J Immunol 33, 1465-70, 2003, *supra).* Phosphorothioate stabilized ODN are cleared from plasma with a half-life of 30-60 minutes (Farman, C.A. & Kornbrust, D.J., Toxicol Pathol 31 Suppl, 119-22, 2003, *supra).* However, in tissues CpG ODN are relatively stable with a half-life of 48 hours (Mutwiri, G.K., et al., J Control Release 97, 1-17, 2004, *supra).* Further, it is noted in the literature that within 60 minutes serum proteases degrade the free peptides below detection levels (Falo, L.D., Jr., et al., Proc Natl Acad Sci USA 89, 8347-50, 1992; Widmann, C., et al., J Immunol 147, 3745-51, 1991, *supra*)*.*

It is observed that immunization leading to CD8⁺ T cell responses of a magnitude comparable to infection with LCMV wild type is provided by administration of both gp33 and CpG in an linearly increasing fashion. It is also observed that immunization using uniform daily doses of gp33 and CpG, although inducing strong CD8⁺ T cell responses, are significantly weaker than dose-escalating stimulation. Furthermore, it is observed that when either one of the vaccine components is delivered as a single dose, the efficacy of immunization is significantly reduced but significant compared to the naïve control.

Similar observations are made in naïve wild type mice that do not receive TCR transgenic cells. C57BL/6 mice immunized with linearly increasing vaccine (gp33 and CpG) doses show significantly enhanced induction of CD8⁺ T cells compared to the other vaccination protocols, which induce barely detectable frequencies of specific CD8⁺ T cells. These results indicate that that, independent of the overall dose, the kinetics of the vaccination is a key parameter of immunogenicity.

### EXAMPLE 11: LINEARLY INCREASING ANTIGENIC STIMULATION ENHANCES PROTECTIVE ANTIVIRAL RESPONSE

An investigation is conducted to determine whether protective antiviral response can be enhanced by increasing antigenic stimulation. In an experiment, female wild type C57BL/6 mice are immunized with fixed cumulative doses of gp33 peptide and CpG (in total 125 µg gp33 and 12.5 nmol CpG) according to different regimens (s1-s4 as described in Example 10) and then challenged with LCMV or a recombinant vaccinia virus expressing the LCMV glycoprotein (vacc-gp) at time points when T cell responses are already in a contraction or memory phase (Kaech, S.M., et al., Nat Rev Immunol 2, 251-62, 2002 *supra*)*.* Protection against both viruses is exclusively dependent on CD8⁺ T cells (Binder, D. and Kundig, T.M., J Immunol 146, 4301-7, 1991; Kundig, T.M. et al., Proc. Natl. Acad. Sci., USA: 93, 9716-23, 1996, *supra*).

Mice (n=4) are immunized with linearly increasing amounts (s4) or with a bolus injection (s1) of gp33 peptide and CpG as described in Example 10. Negative control mice are left untreated (naïve) and positive control mice are infected with LCMV (250 pfu). The mice are bled on day 10 and day 30 for analysis of gp33-specific effector or memory CTLs using gp33-MHC-tetramers and flow cytometry or on day 30 for analysis of IFN-γ-producing CD8⁺ T cells after re-stimulation *in vitro* with gp33. It is observed that linearly increasing doses of peptide (gp33) and CpG induce significantly higher frequencies of IFN-γ-producing effector and memory cells and gp33-tetramer-positive memory (CD44^{hi}) cells than does a single-shot vaccination. On day 30, all mice are challenged by intraperitoneal injection with 250 pfu LCMV. Four days later, viral titers are measured in spleens. On day 30, the mice are challenged intraperitoneally with 250 pfu LCMV. Four or five days later, spleens or ovaries are harvested for determination of LCMV. It is observed that while bolus (s1)-vaccinated mice are not significantly protected against viral replication, linearly increasing vaccination induces significant protection in inhibiting LCMV titers when compared to naïve or bolus-vaccinated mice (p<0.01).

In another set of experiments, C57BL/6 mice are immunized using the different regimes and then challenged intravenously on day 8 or day 24 with 1.5x10⁶ pfu of the recombinant vaccinia virus (vacc-gp). Five days thereafter, vacc-gp replication is determined in ovaries. It is observed that only mice immunized in a dose-escalating fashion are able to mount significantly protective CD8⁺ T cell responses, inhibiting viral replication on orders of magnitude better than the other peptide immunization protocols.

### EXAMPLE 12: LINEARLY INCREASING ANTIGENIC STIMULATION FAVORS PROLONGED T CELL STIMULATION

To test how the kinetics of immunization affect the proliferation of CD8⁺ T cells, mice are injected with a single dose (s1), uniform daily doses (s2) or with linearly increasing doses (s4) of gp33 peptide and CpG as described in Example 10. One group of mice is left untreated as a negative control. To monitor proliferation, all mice receive 10⁷ CFSE-labeled splenocytes from transgenic TCR318 mice intravenously one day before the first immunization. At different time points, lymphocytes are isolated by tail bleeding and analyzed for CD8 expression and CFSE staining by flow cytometry. It is observed that the linearly increasing stimulation markedly prolongs the T cell proliferation relative to the single bolus injection stimulation protocol.

### EXAMPLE 13: LINEARLY INCREASING NUMBERS OF PEPTIDE PULSED DCs ENHANCE CD8⁺ T CELL RESPONSES

To investigate the contribution of different numbers of APCs, C57BL/6 mice are immunized with the same total numbers of peptide-pulsed DCs, but using different kinetics. Bone-marrow derived DCs are loaded with the HPV E7 (aa49-57, RAHYNIVTF, SEQ ID NO:2) peptide, and a total of 1.2×10⁵ cells are injected into the inguinal nodes as a bolus on day one (s1), or the same total number of cells are administered in a linearly increasing (s4) pattern on days one (2×10⁴ cells), three (4×10⁴ cells), and six (6×10⁴ cells). Furthermore, the vaccines are administered intralymphatically in order to ensure a constant total number of DCs available for T cell priming. Naïve mice are used as negative controls. On day 17 and day 22, the frequency of E7-tetramer positive CD8⁺ T cells in peripheral blood is analyzed by flow cytometry, and IFN-γ ELISPOTs are analyzed from spleens). On day 21, three vaccinated mice and ten naïve mice are challenged with the HPV-transformed tumor cell line C3.43. Tumor progression is monitored by caliber measurements (mm) from which tumor volumes are calculated. Survival after challenge is studied in C57BL/6 mice immunized by s.c injection of DCs loaded with the VSV np52 peptide.

It is observed that linearly increasing doses (s4) induce a higher number of antigen-specific CD8⁺ T cells than a bolus injection of the vaccine (s1), as evidenced by both the frequencies of MHC-E7-tetramer positive and of IFN-γ-producing CD8⁺ T cells that are measured on days 17 and 22, respectively. It is also observed that mice vaccinated with the dose-escalating protocol reject a challenge with the HPV-transformed tumor cell line C3.43, while mice vaccinated merely with a single bolus are not protected.

In a further experiment, C57BL/6 mice are immunized by s.c injection of DCs loaded with the VSV np52 peptide. 1.2x10⁵ DCs are given as a bolus on day 1 (s1) or as equal (s2) or dose escalating (s4) doses on days 1, 3, and 6. Naïve mice are used as controls. On day 14, all mice are challenged with dose 10⁶ EL-4 N.1 cells i.p (Kundig et al., J Immunol. 150, 4450-4456, 1993, *supra*). It is observed that the survival of mice immunized with the (s4) protocol of DCs loaded with the VSV np52 peptide is significantly better than mice immunized according to the (s1) or (s2) protocol with DCs given in the uniform numbers on the three days.

The various methods and techniques described above provide a number of ways to carry out the invention. Of course, it is to be understood that not necessarily all objectives or advantages described may be achieved in accordance with any particular embodiment described herein. Thus, for example, those skilled in the art will recognize that the methods may be performed in a manner that achieves or optimizes one advantage or group of advantages as taught herein without necessarily achieving other objectives or advantages as may be taught or suggested herein. A variety of advantageous and disadvantageous alternatives are mentioned herein. It is to be understood that some preferred embodiments specifically include one, another, or several advantageous features, while others specifically exclude one, another, or several disadvantageous features, while still others specifically mitigate a present disadvantageous feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be mixed and matched by one of ordinary skill in this art to perform methods in accordance with principles described herein. Among the various elements, features, and steps some will be specifically included and others specifically excluded in diverse embodiments.

Although the invention has been disclosed in the context of certain embodiments and examples, it will be understood by those skilled in the art that the invention extends beyond the specifically disclosed embodiments to other alternative embodiments and/or uses and modifications and equivalents thereof.

Many variations and alternative elements of the invention have been disclosed. Still further variations and alternate elements will be apparent to one of skill in the art. Among these variations, without limitation, are the specific number of antigens in a screening panel or targeted by a therapeutic product, the type of antigen, the type of cancer, and the particular antigen(s) specified. Various embodiments of the invention can specifically include or exclude any of these variations or elements.

In some embodiments, the numbers expressing quantities of ingredients, properties such as molecular weight, reaction conditions, and so forth, used to describe and claim certain embodiments of the invention are to be understood as being modified in some instances by the term "about." Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

In some embodiments, the terms "a" and "an" and "the" and similar referents used in the context of describing a particular embodiment of the invention (especially in the context of certain of the following claims) may be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is herein deemed to contain the group as modified thus fulfilling the written description of all Markush groups used in the appended claims.

Preferred embodiments of this invention are described herein, including the best mode known to the inventors for carrying out the invention. Variations on those preferred embodiments will become apparent to those of ordinary skill in the art upon reading the foregoing description. It is contemplated that skilled artisans may employ such variations as appropriate, and the invention may be practiced otherwise than specifically described herein. Accordingly, many embodiments of this invention include all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents and printed publications throughout this specification. Each of the above cited references and printed publications are herein individually incorporated by reference in their entirety.

In closing, it is to be understood that the embodiments of the invention disclosed herein are illustrative of the principles of the present invention. Other modifications that may be employed may be within the scope of the invention. Thus, by way of example, but not of limitation, alternative configurations of the present invention may be utilized in accordance with the teachings herein. Accordingly, the present invention is not limited to that precisely as shown and described.
Some embodiments of the invention are disclosed in the following items.

### Items

1. Use of a plurality of sequential doses of an immunogenic composition for stimulating a class I MHC-restricted T cell response in the treatment or prevention of an infectious or neoplastic disease wherein each dose subsequent to an initial dose is greater than the immediately preceding dose.
2. Use of item 1, wherein the sequential doses increase as a linear function of the initial dose.
3. Use of item 1, wherein the sequential doses increase as an exponential function of the initial dose.
4. Use of item 1, wherein the immunogenic composition comprises an immunogen, plus an immunopotentiator or biological response modifier.
5. Use of item 4, wherein the immunopotentiator or biological response modifier is selected from the group consisting of a cytokine, a chemokine, a PAMP, a TLR-ligand, an immunostimulatory sequence, a CpG-containing DNA, a dsRNA, an endocytic-Pattern Recognition Receptor (PRR) ligand, an LPS, a quillaja saponin, and tucaresol.
6. Use of item 3, wherein the exponential function is defined by an exponential factor ≥2ⁿ⁻¹_{.}
7. Use of item 6, wherein the exponential factor is 5ⁿ⁻¹.
8. Use of item 1, wherein the plurality of doses comprises 2 to 6 doses.
9. Use of item 1, wherein the plurality of doses comprises more than 2 doses.
10. Use of item 9, wherein the plurality of doses comprises more than 6 doses.
11. Use of item 1, wherein the last dose is administered within 6 days of the first dose.
12. Use of item 1, wherein an enhanced response is obtained as compared to an immunization utilizing the same cumulative dose without sequentially increasing doses.
13. Use of item 12, wherein the enhanced response comprises an increased number of responding T cells.
14. Use of item 12, wherein the enhanced response comprises increased production of a cytokine.
15. Use of item 14, wherein the cytokine is IL-2 or IFN-γ.
16. Use of item 12, wherein the enhanced response comprises a delay in peak production of an immunosuppressive cytokine.
17. Use of item 16, wherein the immunosuppressive cytokine is IL-10.
18. Use of item 12, wherein the enhanced response comprises an increase in cytolytic activity.
19. Use of item 1, wherein administering the immunogenic composition to the mammal comprises direct delivery to the lymphatic system.
20. Use of item 19, wherein the direct delivery to the lymphatic system comprises intranodal delivery.
21. Use of item 1, wherein administering the immunogenic composition to the mammal comprises subcutaneous, intramuscular, intradermal, transdermal, transmucosal, nasal, bronchial, oral, or rectal administration.
22. Use of item 4, wherein the immunogenic composition comprises an immunogen provided as a protein, peptide, polypeptide, naked DNA vaccine, RNA vaccine, synthetic epitope, or mimotope.
23. Use of item 4, wherein the immunogen stimulates a response to an antigen selected from the group consisting of viral antigens, bacterial antigens, fungal antigens, differentiation antigens, tumor antigens, embryonic antigens, antigens of oncogenes and mutated tumor-suppressor genes, unique tumor antigens resulting from chromosomal translocations, and derivatives thereof.
24. Use of item 23, wherein the antigen is a self-antigen.
25. Use of item 5, wherein the immunopotentiator is a TLR-ligand.
26. Use of item 25, wherein the TLR-ligand is a CpG-containing DNA.
27. Use of item 1, wherein the immunogenic composition comprises a cell.
28. Use of item 27, wherein the cell is a tumor cell.
29. Use of item 27, wherein the cell is an antigen presenting cell.
30. Use of item 29, wherein the antigen presenting cell is a dendritic cell.
31. Use of item 1, wherein the greater dose comprises a greater number of cells.
32. Use of item 31, wherein the greater dose comprises a greater number of epitope-MHC complexes on the surface of the cell.
33. A set of immunogenic compositions comprising an immunogen, plus an immunopotentiator or biological response modifier, wherein the dosages of the individual members of the set are related as an exponential series.
34. The set of item 33, wherein the exponential series of dosages are defined by an exponential factor ≥2ⁿ⁻¹.
35. The set of item 33, wherein the exponential series of dosages are defined by an exponential factor 5ⁿ⁻¹.
36. A kit comprising the set of immunogenic compositions as in item 35 and instructions for administering the composition to a subject in need thereof.
37. The kit of item 36, wherein the immunopotentiator or biological response modifier is selected from the group consisting ofcytokines, chemokines, PAMPs, TLR-ligands, an immunostimulatory sequence, a CpG-containing DNA, a dsRNA, an endocytic-Pattern Recognition Receptor (PRR) ligand, an LPS, a quillaja saponin, and tucaresol.
38. The kit of item 36, wherein the immunogen, and the immunopotentiator or biological response modifier, are each contained in separate containers.
39. The kit of item 36, wherein the immunogen and the immunopotentiator or biological response modifier, are contained in the same container.
40. The kit of item 36, comprising two or more doses of an immunogenic composition each in separate suitable containers.
41. The kit of item 40, wherein the suitable container is a syringe, ampule or vial.
42. A set of syringes comprising sequentially increasing doses of an immunogenic composition wherein each dose subsequent to an initial dose is greater than the immediately preceding dose in each syringe of the set of syringes, and wherein the immunogenic composition comprises an immunogen, and a immunopotentiator or biological response modifier, to enhance a T-cell response in a subject.
43. A set of vials comprising sequentially increasing doses of an immunogenic composition wherein each dose subsequent to an initial dose is greater than the immediately preceding dose in each vial of the set of vials and wherein the immunogenic composition comprises an immunogen, and a immunopotentiator or biological response modifier, to enhance a T-cell response in a subject.
44. Use of a plurality of sequential doses of an immunogenic composition comprising an immunogen, and an immunopotentiator or biological response modifier, in the manufacture of a medicament, wherein each dose subsequent to an initial dose is greater than the immediately preceding dose, for the treatment or prevention of a neoplastic or an infectious disease.
45. A method of stimulating a class I MHC-restricted T cell response in a mammal, said method comprising administering a plurality of sequential doses of an immunogenic composition to the mammal, wherein each dose subsequent to an initial dose is greater than the immediately preceding dose.

## Claims

1. A set comprising a plurality of sequential doses of an immunogenic composition comprising an immunogen and an immunopotentiator or biological response modifier, wherein each dose subsequent to an initial dose is greater than the immediately preceding dose, and wherein the sequential doses of the individual members of the set increase and are related as a linear or exponential series.

2. The set of claim 1, wherein the exponential series of doses are defined by an exponential factor ≥ 2ⁿ⁻¹.

3. The set of any one of the preceding claims, wherein the exponential series of doses are defined by an exponential factor of 5ⁿ⁻¹.

4. The set of any one of the preceding claims, wherein the immunopotentiator or biological response modifier is selected from the group consisting of a cytokine, a chemokine, a PAMP, a TLR-ligand, an immunostimulatory sequence, a CpG-containing DNA, a dsRNA, an endocytic-Pattern Recognition Receptor (PRR) ligand, an LPS, a quillaja saponin, and tucaresol.

5. The set of any one of the preceding claims, wherein the immunogen, and the immunopotentiator or biological response modifier are each contained in separate containers.

6. The set of any one of the preceding claims, wherein the set comprises 2 to 6 doses.

7. The set of any one of claims 1 to 5, wherein the set comprises more than 2 doses.

8. The set of any one of claims 1 to 5, wherein the set comprises more than 6 doses.

9. The set of any one of the preceding claims, wherein the set of immunogenic compositions comprises an immunogen in the form of a protein, peptide, polypeptide, naked DNA vaccine, RNA vaccine, synthetic epitope, or mimotope.

10. The set of any one of the preceding claims, wherein the immunogen stimulates a response to an antigen selected from the group consisting of viral antigens, bacterial antigens, fungal antigens, differentiation antigens, tumor antigens, embryonic antigens, antigens encoded by oncogenes and mutated tumor-suppressor genes, unique tumor antigens resulting from chromosomal translocations, and derivatives thereof.

11. The set of claim 10, wherein the antigen is a self-antigen.

12. The set of any one of the preceding claims, wherein the immunopotentiator is a TLR-ligand.

13. The set of claim 12, wherein the TLR-ligand is a CpG-containing DNA.

14. The set of any one of the preceding claims, wherein the set of immunogenic compositions comprises a cell selected from the group consisting of a tumor cell, an antigen presenting cell, and a dendritic cell.

15. The set of claim 1, wherein a greater dose comprises an increased number of cells or epitope-MHC complexes on the surface of the cell.
